# EUROPÄISCHE PATENTSCHRIFT

(11) **EP 2 314 580 B1**
(45) Veröffentlichungstag und Bekanntmachung des Hinweises auf die Patenterteilung: **09.09.2015**
(21) Anmeldenummer: 10004422.1
(22) Anmeldetag: 27.04.2010
(51) Int. Cl.: C07D 311/00, C07D 311/04, C07D 311/80, C07C 39/14

(54) **Verfahren zur Herstellung von synthetischen Cannabinoiden**
Method for producing synthetic cannabinoids
Procédé de fabrication de cannabinoïdes de synthèse

(30) Priorität: 30.04.2009 DE 102009019322
(43) Veröffentlichungstag der Anmeldung: 27.04.2011
(73) Patentinhaber: The Health Concept GmbH, 60599 Frankfurt am Main (DE)
(72) Erfinder: Steup, Christian, 65719 Hofheim (DE); Herkenroth, Thomas, 83098 Brannenburg (DE)
(74) Vertreter: Pohlmann, Bernd Michael

(56) Entgegenhaltungen:
- US-A- 5 342 971
- PETRZILKA T ET AL: "123. SYNTHESE VON HASCHISCH-INHALTSSTOFFEN", HELVETICA CHIMICA ACTA, VERLAG HELVETICA CHIMICA ACTA, BASEL, CH, Bd. 52, Nr. 123, PART 04, 1. Januar 1969 (1969-01-01), Seiten 1102-1133, XP001088716, ISSN: 0018-019X, DOI: DOI:10.1002/HLCA.19690520427
- CHAN, T.H., CHALY, T.: "A Biomimetic Synthesis of delta1-Tetrahydrocannabinol", TETRAHEDRON LETTERS, Bd. 23, Nr. 29, 1982, Seiten 2935-2938, XP002621301,
- EDERY H ET AL: "STRUCTURE-ACTIVITY RELATIONS IN THE TETRAHYDROCANNABINOL SERIES. MODIFICATIONS ON THE AROMATIC RING AND IN THE SIDE-CHAIN", ARZNEIMITTEL FORSCHUNG. DRUG RESEARCH, ECV EDITIO CANTOR VERLAG, AULENDORF, DE, vol. 22, no. 11, 1 January 1972 (1972-01-01), pages 1995-2003, XP008048340, ISSN: 0004-4172
- LEE Y R ET AL: "Efficient one-pot synthetic approaches for cannabinoid analogues and their application to biologically interesting (-)-hexahydrocannabinol and (+)-hexahydrocannabinol", TETRAHEDRON LETTERS, PERGAMON, GB, vol. 49, no. 20, 12 May 2008 (2008-05-12), pages 3283-3287, XP022613638, ISSN: 0040-4039, DOI: 10.1016/J.TETLET.2008.03.075 [retrieved on 2008-03-20]

## Beschreibung

Das Gebiet der Erfindung ist die organische Synthese, insbesondere ein Verfahren zur Herstellung von Cannabinoiden. Das beschriebene Verfahren ist anwendbar für alle Stereoisomeren und Homologe von Cannabinoiden.

Cannabinoide sind seit der Entdeckung endogener Rezeptoren zunehmend ins Blickfeld der pharmazeutischen Forschung gerückt. Cannabidiol (1a) (die im gesamten Text in Klammern hinter den angegebenen Verbindungen stehenden Ziffern beziehen sich auf die nachfolgend in den Tabellen 1 und 2 abgebildeten Strukturformeln), delta-9-Tetrahydrocannabinol (Dronabinol) (2a) und Nabilon (rac. trans-4b), sowie deren Isomere und Homologe weisen eine Reihe von pharmakologischen Eigenschaften auf, die diese Stoffe therapeutisch interessant machen.

Cannabidiol ist darüber hinaus als Ausgangssubstanz für die Synthese von Dronabinol von besonderer wirtschaftlicher Bedeutung.

Die vorliegende Erfindung macht es sich zur Aufgabe, ein neues Verfahren zur Herstellung der oben genannten Verbindungen mit wenigen Verfahrensschritten und mit einer guten Ausbeute bereit zu stellen.

Die vorliegende Patentanmeldung stellt dazu ein Verfahren zur Herstellung der oben genannten Verbindungen in zwei bzw. drei chemischen Syntheseschritten bereit.

Dabei werden in einem **ersten Schritt ("a")** Verbindungen der allgemeinen Formel III (z.B. Alkylresorcylsäureester (6-Alkyl-2,4-dihydroxybenzoesäure-ester, 5a) mit ungesättigten Kohlenwasserstoffen, Alkoholen, Ketonen (bzw. deren Derivaten wie Enolestern, Enolethern und Ketalen) in hohen Ausbeuten zu den entsprechenden in 3-Stellung substituierten 6-Alkyl-2,4-dihydroxy-benzossäureestern kondensiert.

Beispiele für diese Art von Reaktionen sind u. a. beschrieben worden von Crombie, L. et al. in J. Chem. Research (S) 114, (M), pp.1301-1345 (1977) und wurden dort als säurekatalysierte Terpenylierung bezeichnet.

In einem **zweiten Schritte ("b")** werden die im ersten Schritt erzeugten Zwischenprodukte mit Esterfunktion einer decarboxylierenden Verseifung unterzogen, wodurch die entsprechenden esterfreien Cannabinoide entstehen.

Sofern nötig oder gewünscht wird in einem **dritten Schritt** ("c") eine sauer katalysierte Umlagerung vorgenommen. Diese Isomerisierung kann z. B. der Ringschluss des Pyranrings bei CBD zu Dronabinol sein, aber auch die Umlagerung einer Doppelbindung wie. z. B. die Umwandlung von delta-9- in delta-8-THC oder eine sauer katalysierte Epimeriserung wie die Umlagerung von cis-9-Ketocannabinoiden in die entsprechenden trans-Verbindungen sein.

Die sauer katalysierte Umlagerung c kann, dort wo sie notwendig ist, auch vor dem Verseifungsschritt b erfolgen.

Chan, T.H. und Chaly, T., "A Biomimetic Synthesis of Δ1-Tetrahydrocannabinol", Tetrahedron Letters, Bd. 23, Nr. 29, 1982, Seiten 2935-2938 beschreiben die Synthese von Verbindungen aus Olivetolsäuremethylester und Menta-2,8-dienol in CH2Cl2 mittels BF3-Etherat als Lewissäure und dessen anschließende Verseifung mittels wässriger NaOH unter Zusatz von MeOH, und Decarboxylierung zu Dronabinol. Eine erhöhte Regioselektivität und somit Ausbeute im Kondensationsschritt bei Verwendung des Esters wird ebenfalls beschrieben.

Im Rahmen der vorliegenden Anmeldung ist insbesondere Schritt (b) hervorzuheben, das dieser neu und erfinderisch ist. So wird die Aufgabe der Erfindung dadurch gelöst, dass ein Verfahren vorgeschlagen wird zur Herstellung einer Verbindung der allgemeinen Formel I, insbesondere Ia, Ib und deren Diastereoisomere, 22 oder 35 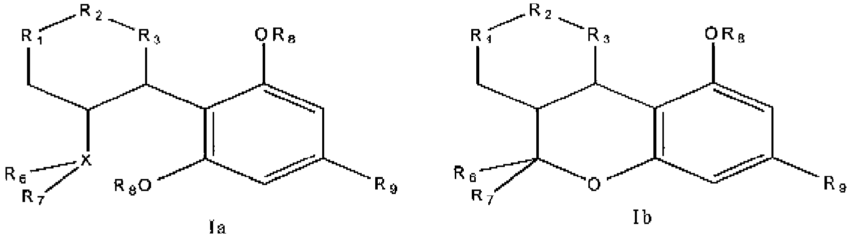 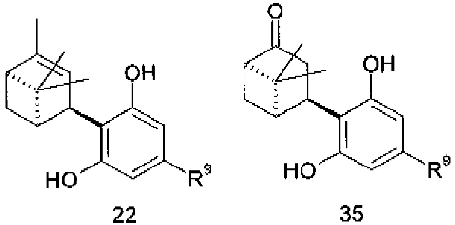 bei dem durch decarboxylierende Verseifung der Verbindung der allgemeinen Formel lla IIb, 21 oder 34, 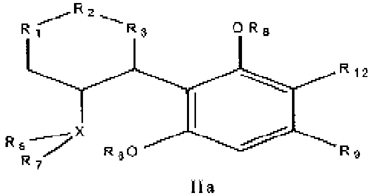 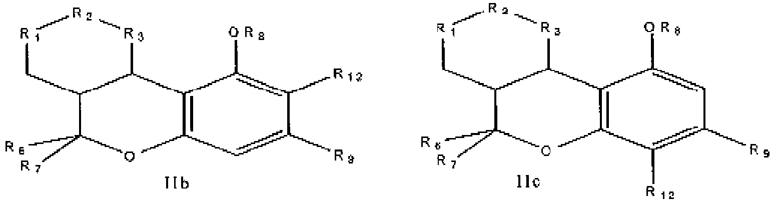 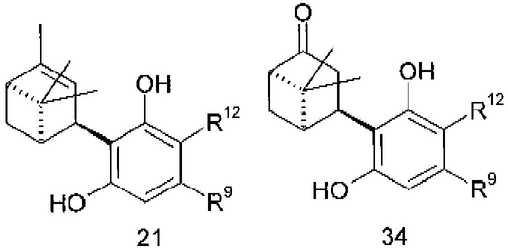 wobei
eine der Bindungen R₁-R₂ oder R₂-R₃ eine C-C Doppelbindung sein kann, und
R₂ entweder C=O oder eine R₁₀-C-R₁₁ Gruppe ist,
wobei
R₁₀ und R11 unabhängig voneinander entweder H, eine niedere C₁-C₄ Alkylgruppe sind, wenn keine Doppelbindung zwischen R1 und R3 vorhanden ist, oder falls eine Doppelbindung zwischen R₁ und R₃ vorhanden ist, eine der Gruppen R₁₀ oder R₁₁ entfällt, und die andere wie oben definiert ist;
X entweder C ist, wenn R6 eine =CH₂ Gruppe ist und R7 eine CH₃ Gruppe ist, oder
X eine CR4 Gruppe ist, wobei R4 H oder niedere Alkylgruppe ist, CH oder eine C-O-R5 Gruppe ist und R5 entweder H, eine C₁ - C₁₆Alkylgruppe oder eine Schutzgruppe ist;
R6 und R7 eine CH₃ Gruppe sind oder wenigstens eine der Gruppen R₆ und R7 eine CH2= Gruppe darstellt und die andere eine CH₃ Gruppe ist,
R₈ eine C₁ - C₁₆ Alkylgruppe, H oder eine Schutzgruppe ist,
R₉ eine C₁-C₁₆-, oder O-C₁-C₁₆-Gruppe ist, wobei C₁-C₁₆ eine gerade oder verzweigte Alkylkette darstellt, die an beliebiger Stelle eine oder mehrere Doppel- oder Dreifachbindungen aufweist oder Substituenten wie Deuterium- oder Halogen-Atome, Phenyl-, substituierte Phenyl-, Cycloalkyl-, Nitril-, Alkoxy-Gruppen oder eine Ketogruppe aufweisen kann,
R₁₂ eine CO₂-R₁₃-Gruppe ist, und
R₁₃ eine C₁-C₁₆-Alkylgruppe oder eine Schutzgruppe ist,
Cannabinoide erhalten werden

Um die Ester-Zwischenprodukte des Kondensationsschritts "a" in die Endprodukte überzuführen, ist es notwendig, die Estergruppe der Kondensationsprodukte der ersten Stufe zu verseifen und zu decarboxylieren (Schritt "b").

Bei den Verbindungen vom Typ 16 wie z. B. Cannabidiolcarbonsäureester entstehen durch saure Behandlung in großem Maße unerwünschte Nebenprodukte wie delta-8-Tetrahydrocannabinol und iso-Tetrahydrocannabinole (Israel Journal of Chemistry, Vol.6, 1968, 679-690).

Dies gilt auch für die Herstellung von delta-9-Tetrahydrocannabinol aus den Estern der delta-9-Tetrahydrocannabinolsäure A oder B (Formelbilder 17a und 18a).

Dies gilt selbstverständlich auch für die Homologen der genannten Cannabinoide.

Es gilt deshalb, dass auch die direkte Synthese von delta-9-Tetrahydrocannabinol aus delta-9-Tetrahydrocannabinolsäureestern durch Verseifung sich wegen der Tendenz der Doppelbindung in Position "8" zu wandern, sich nicht im sauren Milieu durchführen lässt.

Daher bietet sich die alkalische Verseifung an (Schritt "b").

Auch im Falle der Ketocannabinoide wie Nabilon, dessen Stereoisomeren und deren Homologen lassen sich die hier unter "b" beschriebenen Verfahren zur Verseifung und Decarboxylierung anwenden und liefern überlegene Ausbeuten an den erwünschten Produkten.

Durch Epimerisierung der Estervorstufen mit Säuren bilden sich aber weniger Nebenprodukte als bei der Behandlung der esterfreien Endprodukte.

Die alkalische Verseifung der Estergruppe mit nachfolgender Decarboxylierung erlaubt es Cannabinoide vom Typ des Verbenylolivetolat (Formelbild 21a) oder Verbindungen vom Typ 35 (Formelbild 35) ohne Isomerisierung herzustellen.

Dadurch wird der bei der Synthese von Cannabinoiden mit Alkylresorcylsäureestern gegenüber einer Synthese mit Alkylresorcinolen erzielte Vorteil einer höheren Regioselektivität sowie die erhöhte Stabilität der Zwischenprodukte mit Estergruppe optimal genutzt.

Bei Verbindungen, welchen im Sauren zu Isomerisierung (Ringschluss und/oder Wanderung der Doppelbindung) neigen, wie CBD-Carbonsäureestem ([Formelbild 16], R1 = n-C5H11) und CBD ([Formelbild 1], R1 = n-C5H11) oder den Estern der Tetrahydrocannabinolsäuren A und B [17a] und [18a] macht erst ein entsprechend optimiertes Verfahren zur Verseifung und Decarboxylierung der Esterzwischenstufen den eingangs beschriebenen Syntheseweg wirtschaftlich interessant.

Allerdings entstehen aufgrund der Acidität der phenolischen Hydroxylgruppen bei der Reaktion der Esterzwischenstufen mit Alkalien Phenolat-Anionen, welche gegen einen weiteren Angriff von Hydroxid - Anionen relativ resistent sind.

Das in der Literatur beschriebene Verfahren Ester vom Typ [16] (Petrzilka et al, Helv. Chim. Acata 52 (1969), 1102-1134) oder [18] (Herlt at al, US-Pat. 5342,971) mit Alkalien in Methanol zu kochen, lieferte auch nach sehr langen Reaktionszeiten von mehreren Tagen in unseren Händen weitgehend unverändertes Ausgangsmaterial und nur geringe Ausbeuten an dem erwünschten Produkt. Es ist daher für eine wirtschaftliche Synthese von Arzneiwirkstoffen und ihren Vorstufen nicht brauchbar.

Im technischen Maßstab ist die Kesselbelegungszeit ein erheblicher Faktor, der über die Wirtschaftlichkeit eines Verfahrens entscheidet.

Auch macht die Anwesenheit unreagierten Ausgangsmaterials aufwändige Reinigungsschritte wie Chromatographie nötig in den Fällen, in denen ansonsten das Produkt durch weniger kostspielige Verfahren wie Destillation und Kristallisation rein darstellbar ist.

Es wurde deshalb nach einem wirtschaftlichen Verfahren gesucht, das innerhalb weniger Stunden zu einem vollständigen Umsatz des Ausgangsmaterials führt und die gewünschten verseiften und decarboxylierten Produkte mit guten Ausbeuten liefert.

Wir fanden nun, dass sich die Estervorstufen der Cannabinoide in hervorragenden Ausbeuten und praktisch ohne Bildung von Nebenprodukten zu den entsprechenden Endprodukten verseifen und decarboxylieren lassen, wenn eines der folgenden Verfahren (hier unter Reaktionsschritt "b" zusammengefasst) angewendet wird:
1. Ein Druckverfahren, welches die Anwendung erhöhter Temperaturen bei gleichzeitiger Verwendung niedrig siedender Lösungsmittel wie niederen Alkoholen und deren Mischung mit Wasser erlaubt.
   Geeignete Lösungsmittel für das Druckverfahren sind niedere Alkohole mit einem bis zu fünf Kohlenstoffatomen, Ammoniak, sowie deren Mischungen untereinander und mit Wasser. Ebenso kann reines Wasser mit einem Phasentransferkatalysator oder Emulgator zur Anwendung kommen.
2. Ein druckloses Verfahren, welches eine Reaktionsführung in einem "offenen" System erlaubt.
   Geeignete Lösungsmittel für das drucklose Verfahren sind mit Wasser mischbare Lösungsmittel mit einem Siedepunkt über 100°C bei Normaldruck, wie zum Beispiel Dimethylformamid, Dimethylsulfoxid, Sulfolan, Furfurol, Di- und Tetrahydrofurfurol, 2-Methoxytetrahydrofuran, Hexamethylenphosphorsäutetriamid, Acetamid und andere Amide mit bis zu 12 Kohlenstoffatomen, Tetramethyl-hamstoff, Ethylenglykol sowie seine Mono- und Bis-Ether, Ethanolamin, Ethylendiamin, Propylenglykol und seine Ether mit bis zu 20 Kohlenstoffatomen, Glycerin und Glycerinether mit zu 30 Kohlenstoffatomen, 1,2-Butandiol, 1,3-Butandiol, 1,4-Butandiol, Diethylenglykol und seine Ether, Triethylenglykol und seine Ether, Polyethylenglykol und Polyvinylpyrrolidon, sowie deren Mischungen untereinander und mit Wasser.
   Für beide Verfahren gilt:
   Zur alkalischen Verseifung geeignete Basen sind die Hydroxide von Alkali- und Erdalkalimetallen sowie von quaternären Ammoniumsalzen, die Carbonate, Hydrogencarbonate, Carboxylate mit einem bis zu 30 Kohlenstoffatomen, Phenolate, Phosphate, Phosphite, Sulfide, Hydrogensulfide, Mercaptide von einem bis zu 30 Kohlenstoffatomen, Sulfite, Hydrogensulfite, Cyanide von Alkalimetallen und quaternären Ammoniumsalzen mit vier bis 48 Kohlenstoffatomen.
   Ebenso geeignet sind Ammoniak und organische Amine (auch Pyridinbasen) mit einem bis zu 36 Kohlenstoffatomen, sowie deren Salze, ferner auch Borate wie Natriumtetraborat (Borax) und andere basische Salze,
3. Die Anwendung von Katalysatoren (mehrwertige Kationen, sowie feine verteilte Übergangsmetalle und deren Salze), welche die Decarboxylierung der als Zwischenprodukte nach der Verseifung entstehenden Säuren (Cannabinoid-Carbonsäuren) beschleunigen und damit diese Säuren aus dem Reaktionsgleichgewicht entfemen.

Geeignete Katalysatoren sind fein verteilte Übergangsmetalle sowie die Salze von Übergangsmetallen, z. **B.** Edelstahlpulver oder Silberpulver.

Der Vorteil des Druckverfahrens liegt in der leichten Abtrennbarkeit niedrig siedender Lösungsmittel von den Reaktionsprodukten durch Destillation, was das Recycling des Lösungsmittels erleichtert und das Verfahren umweltfreundlicher macht.

Der Vorteil des drucklosen Verfahrens liegt in dem geringeren apparativen Aufwand, der sich durch die Reaktionsführung in einem offenen System gegenüber einem Druckbehälter ergibt.

Um die Zwischenprodukte IIa, IIb, IIc, 21 und 24 herzustellen werden in einem **ersten Schritt a** Alkylresorcylsäureester (6-Alkyl-2,4-dihydroxybenzoesäure-ester) (Formelbild 5) mit ungesättigten Kohlenwasserstoffen, Alkoholen, Ketonen (bzw. deren Derivaten wie Enolestern, Enolethern und Ketalen) in hohen Ausbeuten zu den entsprechenden in 3-Stellung substituierten 6-Alkyl-2,4-dihy-droxy-benzoesäureestern kondensiert.

In einem **zweiten Schritte b** werden die im ersten Schritt erzeugten Zwischenprodukte mit Esterfunktion einer decarboxylierenden Verseifung unterzogen, wodurch die entsprechenden esterfreien Cannabinoide entstehen.

Sofern nötig wird in einem **dritten Schritt c** eine sauer katalysierte Umlagerung vorgenommen. Diese Isomerisierung kann z. B. der Ringschluss des Pyranrings bei CBD zu Dronabinol sein, aber auch die Umlagerung einer Doppelbindung wie. z. B. die Umwandlung von delta-9- in delta-8-THC oder eine sauer katalysierte Epimerisierung wie die Umlagerung von cis-9-Ketocannabinoiden in die entsprechenden trans-Verbindungen.

Die sauer katalysierte Umlagerung c kann, dort wo sie notwendig ist, auch vor dem Verseifungsschritt b erfolgen.

Es können also auch die hinsichtlich der Aufbau des Kohlenwasserstoff Skeletts fertigen Estervorstufen der decarboxylierenden Verseifung unterzogen werden.

R¹ ist dabei eine gerade oder verzweigte Alkylkette oder Alkoxykette von einem bis zu 16 Kohlenstoff (C)-Atomen, welche an beliebiger Stelle Doppelbindungen, Dreifachbindungen oder weitere Substituenten wie Deuterium-Atome, Phenyl-, substituiertes Phenyl-, Cycloalkyl, Nitril-, Alkoxy und Ketogruppen aufweisen kann.

R² ist eine Carboxyl-Schutzfunktion *(Definition analog zu Herlt* US-Patent 5,342,971 *p.4)* von einem bis zu 16 Kohlenstoffatomen, typischerweise eine Alkylfunktion oder eine substituierte Alkylfunktion wie Benzyl (Phenylmethyl-), Diphenylmethyl- oder solchen in 2-Stellung substituierten Alkytresten von einem bis 16 C-Atomen wie (i) niederem Alkoxy-z.B. 2-Methoxyethyl , 2-Ethoxyethyl, (ii) niederem Alkylthiio wie z: B. 2-Methylthioethyl-und 2-Ethylthioethyl, (iii) Halogen wie 2,2,2-Trichlorethyl, 2-Bromethyl und 2-Chlorethyl, (iv) einer oder zwei Phenylgruppen (substituiert oder unsubstituiert) substituierten Alkylgruppen; sowie Aroylgruppen wie Phenacyl.

Tabelle l zeigt Beispiele der Verbindungen, die durch das erfindungsgemäße Verfahren erhalten werden:

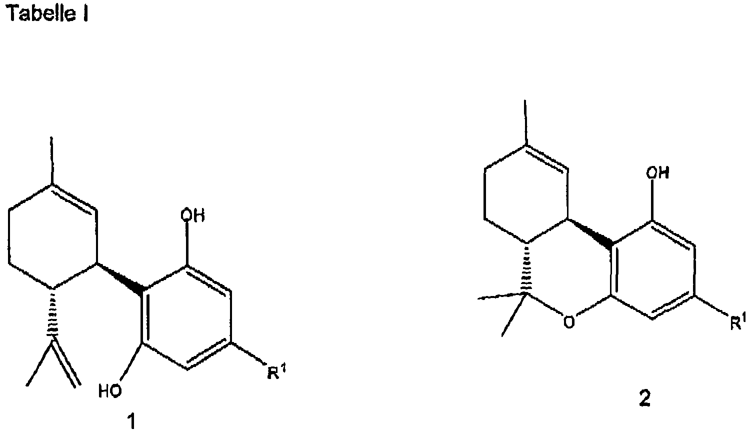

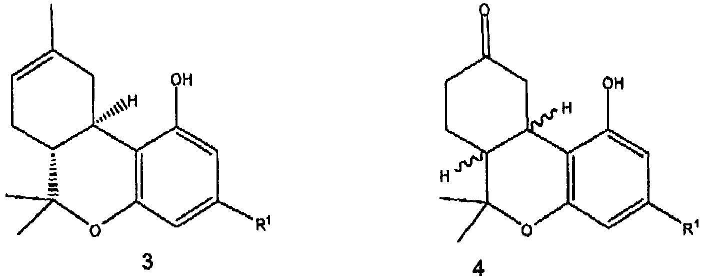

wobei z.B.:
a: R¹ = n-C₅H₁₁ (entspricht 1)
(-)-CBD entspricht 1 a:
2-((1R,6R)-3-Methyl-6-(prop-1-en-2-yl)cyclohex-2-enyl)-5-pentylbenzen-1,3-diol(-)-delta-9-THC entspricht 2a:
(6aR,10aR)-6,6,9-Trimethyl-3-pentyl-6a,7,8,10a-tetrahydro-6H-benzo[c]chromen-1-ol-delta-8-THC entspricht 3a:
(6aR,10aR)-6,6,9-Trimethyl-3-pentyl-6a,7,10,10a-tetrahydro-6H-benzo[c]chromen-1-ol
b: R1 = 1,1-Dimethylheptyl-
Nabilon = racemisches trans entspricht 4b:
   racemisches trans-1-Hydroxy-6,6-dimethyl-3-(2-methyloctan-2-yl)-7,8,10,10a-tetrahydro-6H-benzo[c]chromen-9(6aH)-on

Durch Verwendung geeigneter Reaktionspartner aus Tabelle ll lassen sich auf die oben ausgeführte Art und Weise Cannabinoidcarbonsäureester aufbauen.

Tabelle ll mit den Verbindungen (5) bis (15) gibt eine Übersicht möglicher zur Kondensation (Schritt a) verwendbarer ungesättigter Kohlenwasserstoffe, Alkohole und Ketone (bzw. deren Derivate wie Enolester, Enolether und Ketale).

Eine Ketofunktion kann dabei als Enolether, Enolester oder Ketal geschützt sein, wobei R³ und R⁴ geradkettige, verzweigte oder zyklische organische Gruppen mit bis zu 16 Kohlenstoffatomen oder siliciumorganische Reste mit bis zu 16 Kohlenstoffatomen sein können.

R³ und R⁴ können auch miteinander verbrückte geradkettige oder verzweigte Kohlenwasserstoffreste sein mit bis zu 16 C-Atomen wie z. B. -(CH₂)n-, -CH₂(CCH₃)₂CH₂-.

Die Gruppen R⁵ und R⁶ können dabei [Formeltyp (6) bis (15)] Wasserstoff (H) oder eine alkoholische Schutzfunktion wie ein geradkettiges, verzweigtes oder zyklisches Alkyl-, Acyl- oder ein siliciumorganischer Rest mit bis zu 16 C-Atomen sein.

Besondere Betonung verdient die Tatsache, dass im Falle der aufgeführten Verbindungen auch Ether und Ester (R⁵ bzw. R⁶ = geradkettiges , verzweigtes oder zyklisches Alkyl-, Acyl-, Siliciumorganyl- mit jeweils bis zu 16 C-Atomen) unter den Bedingungen der sauren Terpenylierung (Reaktionsschritt "a") wie die entsprechenden freien Alkohole (R⁵ bzw. R⁶ = H) reagieren können.

Werden im Menthan - Strukturteil am C-4 optisch aktiv substituierteTerpene eingesetzt, so lassen sich optisch aktive Cannabinoide als Endprodukte darstellen (vgl. auch T. Petrzilka et al,: Helv. Chim. Acta Vol. 52 (1969) 1102 -1134).

Das Gleiche gilt für die Bicycloheptane -und Bicycloheptene wie Verbenol (8a; R⁵ = H), Apoverbenon (14) oder Verbindungen vom Typ (15), wenn bezüglich der Konfiguration an den Brückenkopfatomen C1 und C5 eindeutig definierte Verbindungen eingesetzt werden.

Diese kondensieren mit (5) unter Beibehalt der absoluten Konfiguration an C4 bzw. C5 und lassen so optisch aktive Esterzwischenprodukte und Cannabinoide herstellen.

**Tabelle II (Reaktionspartner für Syntheseschritt a):**

| | | |
|---|---|---|
| 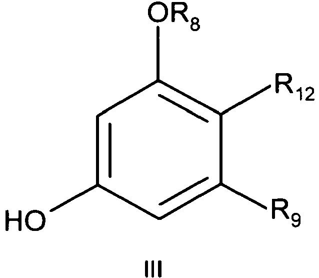 | | |
| 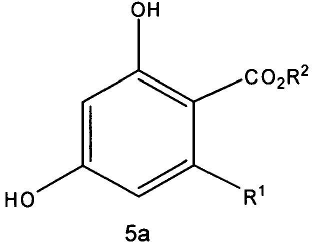 | 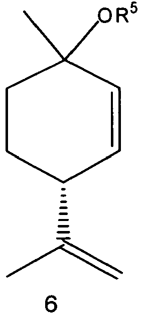 | |
| 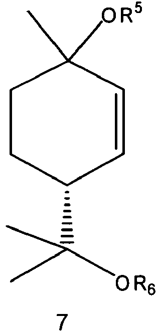 | 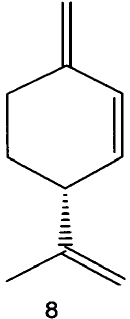 | 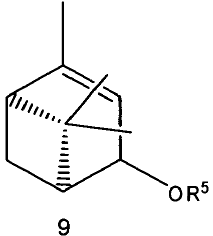 |
| 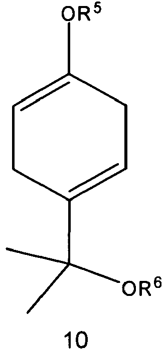 | 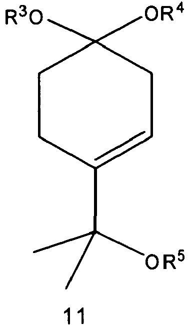 | 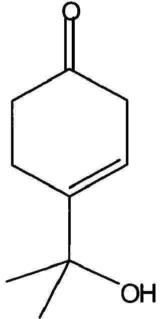 |
| "4-(2-Hydroxypropan-2-yl)cyclohex-3-enon" | | |
| 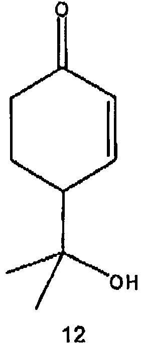 | 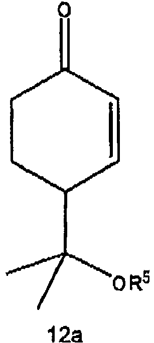 | |
| 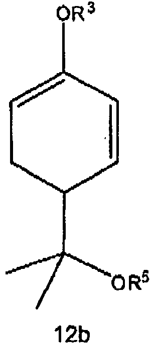 | 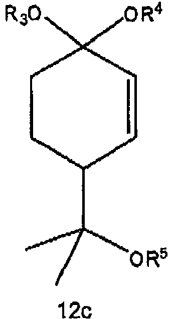 | |
| 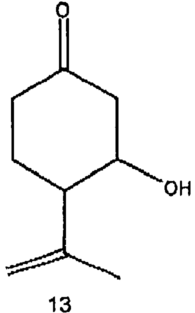 | 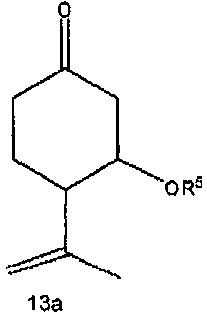 | |
| 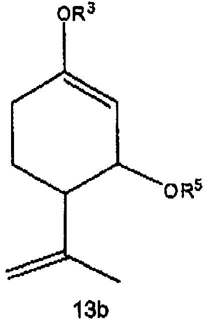 | 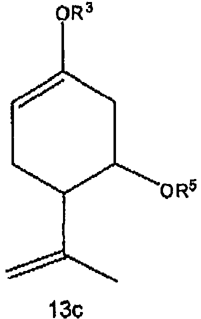 | |
| 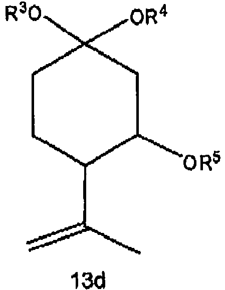 | | |
| 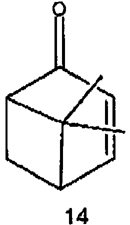 | 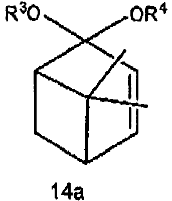 | |
| 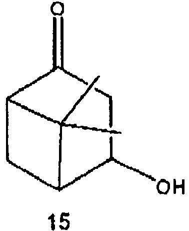 | 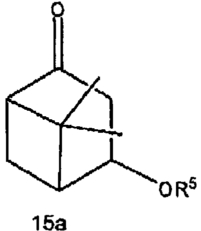 | |
| 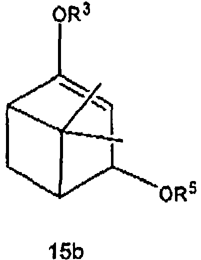 | 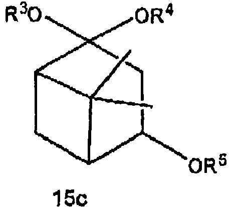 | |

### Beispiele zu den Formeln der Tabelle ll:

6-Alkyl-2,4-dihydroxybenzoesäure-ester (Alkylresorcylsäureester) (5a): 5a entspricht der allgemeinen Formel lll, wenn R8 H bedeutet und R1 und R2 den Substituenten R9 und R12 der Formel III (R8 eine C₁ - C₁₆Alkylgruppe, H oder eine Schutzgruppe ist, R9 eine C1-C16-, oder O-C₁-C₁₆-Gruppe ist, wobei C₁-C₁₆ eine gerade oder verzweigte Alkylkette darstellt, die an beliebiger Stelle eine oder mehrere Doppel- oder Dreifachbindungen aufweist oder Substituente wie Deuterium- oder Halogen-Atome, Phenyl-, substituierte Phenyl-, Cycloalkyl-, Nitril-, Alkoxy oder eine Ketogruppe aufweisen kann, R12 eine CO₂-R13-Gruppe ist, und R13 H, C₁-C₁₆ eine Alkylgruppe oder eine Schutzgruppe ist) entsprechen.
cis- und trans-p-Mentha-2,8-dien-1-ol (6a, R⁵ = H)
p-Mentha-2-en-1,8-diol (7a, R⁵ R⁶ = H)
Menthatrien (8)
(-)-trans-Verbenol (9a, R⁵ = H)

Aus 4-Methocyacetophenon durch Grignard und nachfolgende Birch-Reduktion zugängliche Strukturen vom Typ 10 wie z. B. 10a:
2-(4-Methoxycyclohexa-1,4-dienyl)propan-2-ol (10a; R⁵ = CH₃, R⁶ = H)

Strukturen vom Typ 11 wie z. B,: 2-(4,4-Dimethoxycyclohex-1-enyl)propan-2-ol (11a, R³ = R⁴ = CH₃; R⁵ = H); diese lassen sich als ein maskiertes "4-(2-Hydroxypropan-2-yl)cyclohex-3-enon auffassen".

4-(2-Hydroxypropan-2-yl)-cyclohex-2-enon (12) und seine maskierten Formen 12a bis 12c, 3-Hydroxy-4-(prop-1-en-2-yl)-cyclohexanon (13) und seine maskierten Formen 13a bis 13d.

6,6-Dimethylbicyclo[3.1.1]hept-3-en-2on (Apoverbenon) (14) und seine maskierten Formen wie z. B. 6,6-Dimethyl-2,2-diacetoxy-3-norpinen (13a, R³ = R⁴ = COCH₃)

4-Hydroxy-6,6-dimethylbicyclo[3.1.1]heptan-2on (15) und seine maskierten Formen wie z. B. 6,6-Dimethyl-2,4-diacetoxy-2-norpinen (15 b, R³ = R⁵ = COCH₃)

Dabei lassen sich mit den Verbindungen 6 bis 9 Cannabinoide vom Typ 1 bis 3 aufbauen, während mit den Verbindungen 10 bis 15 9-Ketocannabinoide vom Typ 4 wie z. B. Nabilon aufgebaut werden können.

Die durch die Reaktion a entstehenden Zwischenprodukte weisen eine Esterfunktion CO₂R² auf und werden im Folgenden als "Esterzwischenpradukte" bezeichnet.

In einem darauf folgenden Reaktionsschritt werden die Esterzwischenprodukte in die entsprechenden esterfreien Cannabinoide umgewandelt, welche an Stelle der Esterfunktion eine Wasserstofffuriktion tragen.

In einigen Fällen ist zur Synthese des gewünschten Endproduktes noch eine sauer katalysierte Umlagerung (Reaktionsschritt c) notwendig.

Diese Umlagerung kann eine Isomerisierung oder als Spezialfall davon eine Epimerisierung sein und kann sowohl vor wie auch nach dem Reaktionsschritt b durchgeführt werden.

Die sauer katalysierte Umlagerung c und die saure Terpenylierung können in einigen Fällen auch vorteilhaft als "Eintopfverfahren" durchgeführt werden, so dass die umgelagerten Zwischenprodukte der decarboxylierenden Verseifung b unterzogen werden können.

Werden z. B. Verbindungen vom Typ 1 nach Methode a mit (+)-cis- oder trans-Menthadienol 6a (R⁵ = H) oder seinen Estern kondensiert, so entstehen in hervorragenden Ausbeuten Esterzwischenprodukte vom Typ 16 wie z. B. der Cannabidiolsäuremethylester 16a (R¹ = n-C₅H₁₁; R² =CH₃).

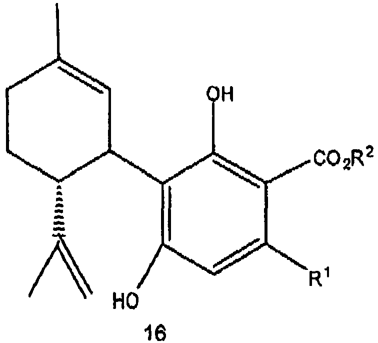

Durch decarboxylierende Verseifung lässt sich daraus Cannabidiol (1a;. R¹ = n-C₅H₁₁) herstellen und in einem nachfolgenden sauer katalysierten Isomerisierungsschritt c (-)-delta-9-Tetrahydrocannabinol (2a;. R¹ = n-C₅H₁₁).

Aus letzterem lässt sich durch verlängerte Einwirkungszeit des sauren Katalysators delta-8-Tetrahydrocannabinol (3a;. R¹ = n-C₅H₁₁) herstellen

Man kann den Isomerisierungsschritt aber auch vor der decarboxylierenden Verseifung c durchführen.

So erhält man aus dem Kondensationsprodukt (16a) von Olivetolsäuremethylester (5a R¹ = n-C₅H₁₁; R² = CH₃) mit Menthadienol 6a (R⁵ = H) oder seinen Estern die beiden Stellungsisomeren delta-9-Teirahydrocannabinolsäureester-A 17 und delta-9-Tetrahydrocannabinolsäureester-B 18 (jeweils R¹ = n-C₅H₁₁: R²= CH₃).

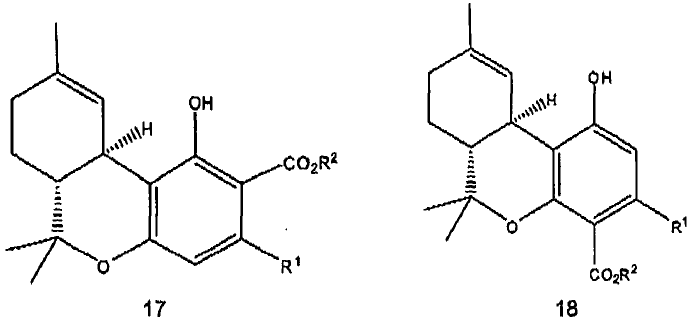

Verlängerte Einwirkungszeit des Katalysators führt auch hier zur Umtagerung der Doppelbindung in die Position 8:

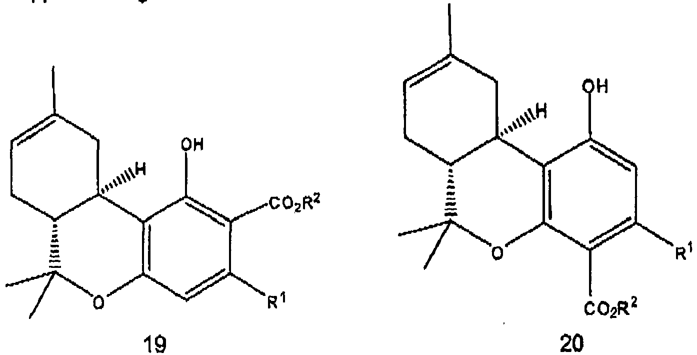

So entsteht aus dem delta-9-Tetrahydrocannabinolsäureester-A (17) der delta-8-Tetrahydrocannabinotsäureester-A (19) und aus delta-9-Tetrahydrocannabinolsäureester-B (18) der delta-8-Tetrahydrocannabinolsäureester-B (20) (jeweils R¹ = n-C₅H₁₁; R² = CH₃).

Aus den Verbindungen (17) und (18) lassen sich nach Methode b Verbindungen vom Typ (2) erhalten.

Aus den Verbindungen (19) und (20) lassen sich nach Methode b Verbindungen vom Typ (3) erhalten.

Die gleichen Reaktionen wie mit (6) lassen sich mit (7) und (8) durchführen.

(-)-trans-Verbenol (9a; R⁵ = H) und seine Ester ließen sich mit Olivetolsäuremethylester (5a; R¹ = n-C₅H₁₁; R² = CH₃) zu "para"- Verbenyl-olivetolat (21a; R¹ = n-C₅H₁₁; R² = CH₃) (Reaktion "a") kondensieren und daraus säurekatalysiert (Reaktion "c") delta-8-Tetrahydrocannabinol-säure- A -methylester (19a)] und delta-8-Tetrahydrocannabinolsäure- B - methylester herstellen (20a) (Crombie bezeichnet diese als delta-^{1,6}- Tetrahydrocannabinolsäure- A und -B -methylester).

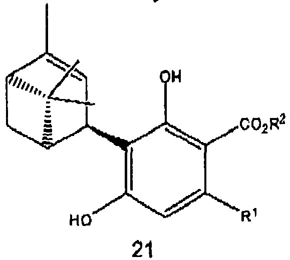

Die hier beschriebene Verseifung und Decarboxylierung (Verfahren b) von Verbenylolivetolat erlaubt es Verbenyl-olivetol (22a; R¹ = n-C₅H₁₁)) herzustellen und daraus mittels säurekatalysierter Isomerisierung (Verfahren ""c") delta-8- oder delta-9-THC herzustellen.

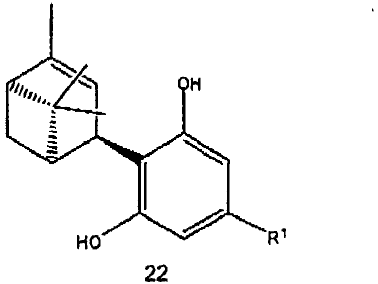

Aus Olivetolsäuremethylester, (R¹ = n-C₅H₁₁, R² = CH₃) und Geraniol lässt sich durch saure Kondensation Cannabigerolsäure-methylester (Methylcannabigerolat) und daraus aus den unter b beschriebenen Verfahren Cannabigerol (CBG) herstellen. Aus Citral kann man so die Cannabichromensäuremethylester A und B herstellen. Beide ergeben nach den Verfahren b Cannabichromen.

Vergleiche zum Beispiel die Herstellung von Cannabigerol über Cannabigerolsäureester und von Cannabichromen über Cannabichromensäureester-A bzw. -B auf Seite 19 und 20.

Besonders eignet sich der hier angegebene Syntheseweg zur Darstellung der niederen Homologen von CBD und Tetrahydrocannabinol, wie zum Beispiel Cannabidivarol CBDV, R¹ = n-C₃H₇ und delta-9-Tetrahydrocannabivarol THCV R¹ = n-C,₃H₇, da durch die Estergruppe im Kondensationsschritt "a" die Bildung von Stellungsisomeren zurückgedrängt wird ( R¹ = n-C₃H₇).

Auch im Falle der Ketocannabinoide wie z. B. Nabilon, dessen Stereoisomeren und deren Homologen lassen sich die hier beschriebenen Verfahren zur Kondensation "a" von Alkoholen, Ketonen (bzw. deren Derivaten wie Enolethem, Enolestern und Ketalen), Carbonsäuren und Ester mit Verbindungen vom Typ lll vorteilhaft anwenden und liefern nach erfolgter Verseifung und Decarboxylierung (Verfahren "b") überlegene Ausbeuten an den erwünschten Produkten. 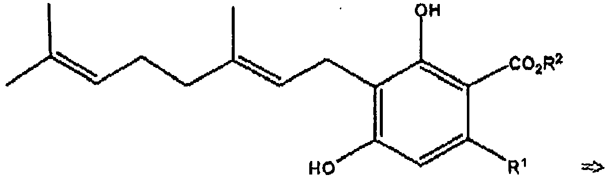 Cannabigerolcarbonsäuremethylester R¹ = n-C₅H₁₁, R² = CH₃ 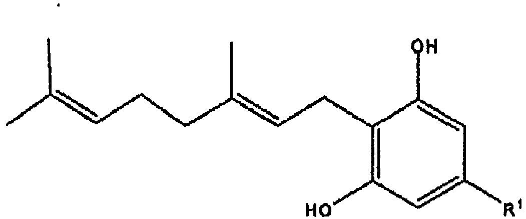 Cannabigerol R¹ = n-C₅H₁₁ ***** 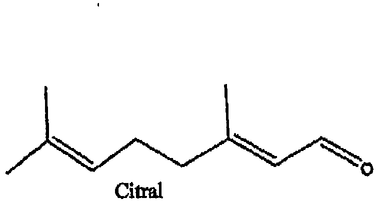 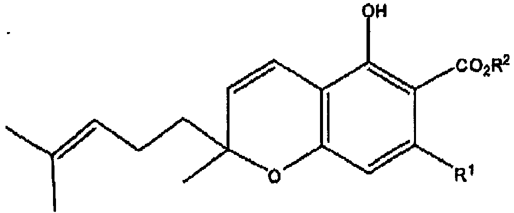 Cannabichromensaüremethylester-A: R¹ = n-C₅H₁₁, R² = CH₃ 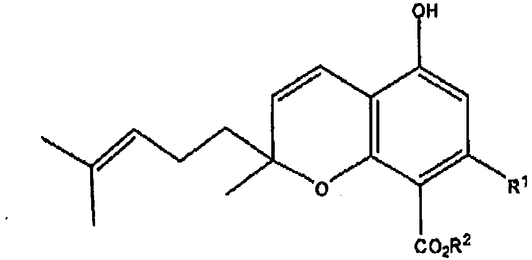 Cannabichromensäuremethylester-B: R¹ = n-C₅H₁₁, R² = CH₃ 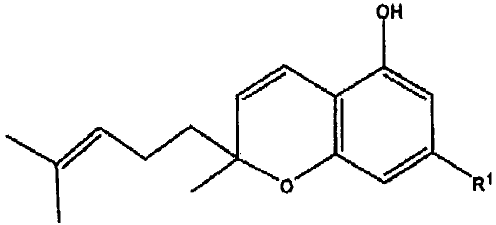 Cannabichromen R¹ = n-C₅H₁₁ *****

Mit 5 kondensieren

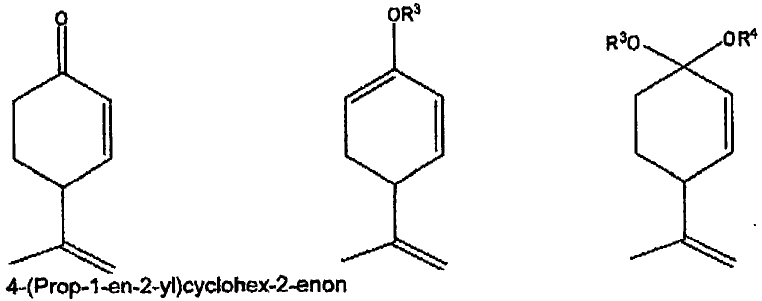

Bringt man weiter z. B. Verbindungen vom Typ (5) mit 2-(4-Methoxy-1,4-cyclohexadienyl)-2-propanol (10a; R⁵ = R⁶ = CM₃) nach Verfahren "a" zur Reaktion, entstehen die Estervorstufen von Nabilon und seinen Homologen in cis-Form:(23-A), [23] als racemische Mischung der Stellungsisomeren Ester A (23-A und 24-A) und B (23-B und 24-B)

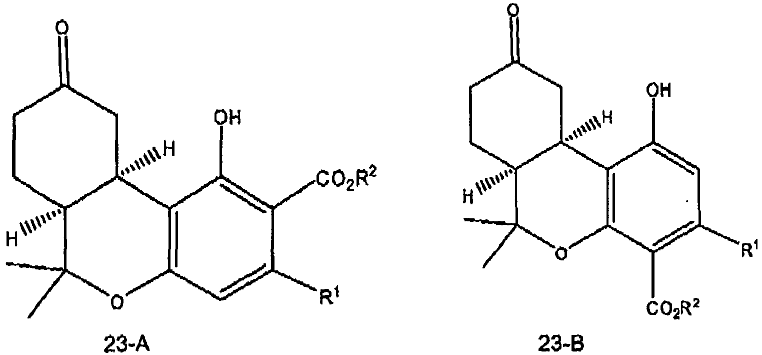

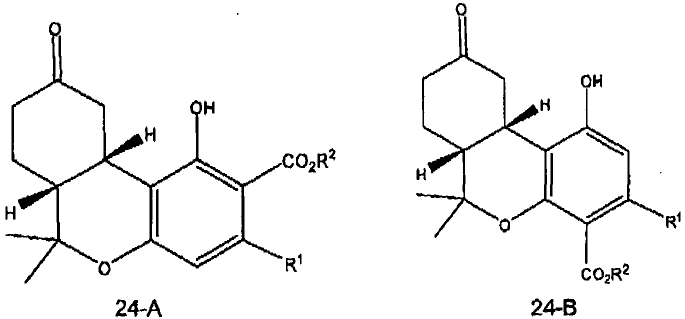

Je nach verwendetem Katalysator und Lösungsmittel entstehen im Kondensationsschritt auch Acetale (25-A) und (25-B) oder (26-A) und (26-B).

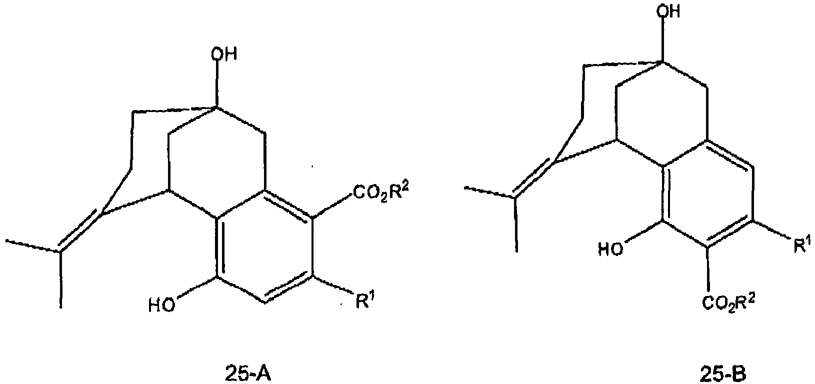

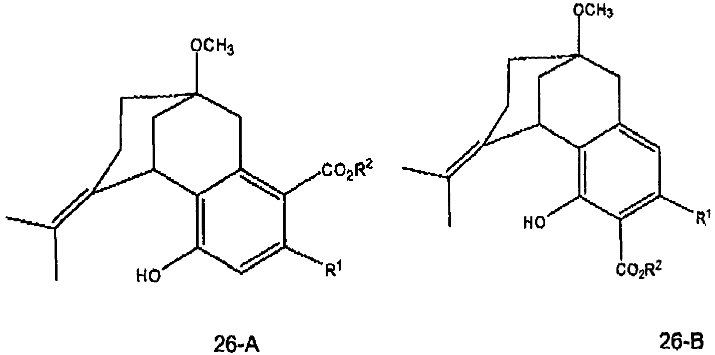

Aus 23-A und 24-A wird durch sauer katalysierte Epimerisierung das Racemat der trans-Ester 27-A und 28-A (27-A und 28-A als racemisches Gemisch):

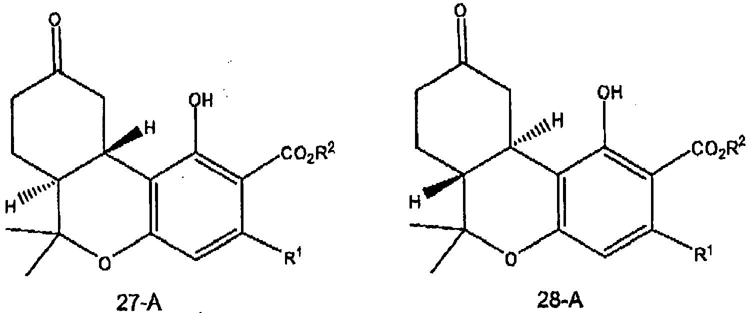

Aus 23-B und 24-B wird durch sauer katalysierte Epimerisierung das Racemat der trans-Ester 27-B und 28-B (27-B und 28-B als racemisches Gemisch):

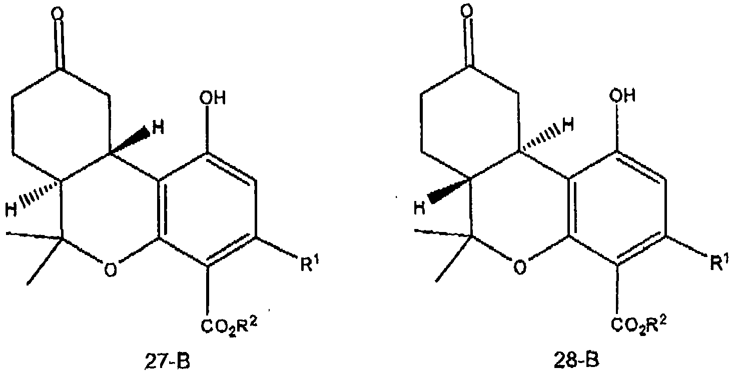

Aus den Acetalen 25 und 26 wird durch sauer katalysierte Umlagerung das Gemisch der cis-Ester 23 und 24, welche sich sauer weiter in die trans-ester-Ester 27 und 28 umlagern lassen.

Durch alkalische decarboxylierende Verseifung (Schritt b) entstehen aus den trans-Verbindungen 27 und 28 racemische Verbindungen vom Typ trans-(4), wie z. B. Nabilon (R¹ = 1,1-Dimethylheptyl)

Man kann die cis-Verbindungen 23 und 24 wie auch die Acetale 25 und 26 auch zuerst der decarboxylierenden Verseifung unterziehen und die Umlagerung analog Archer et al.: (J. Org. Chem. Vol. 42 pp. 1177-2284) an den entsprechenden esterfreien cis-Verbindungen praktizieren.

Aus 23-A und -B wird somit 29:

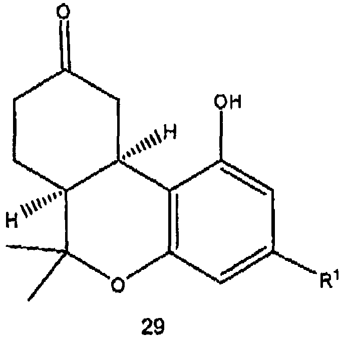

Aus 24-A und -B wird somit 30:

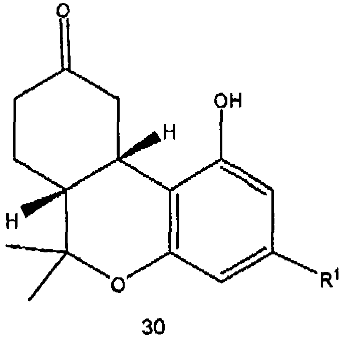

Aus den Acetalen 25 werden Verbindungen vom Typ 31:

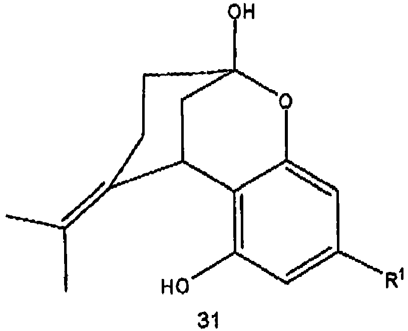

Aus den Acetalen vom Typ 26 werden Verbindungen vom Typ 32:

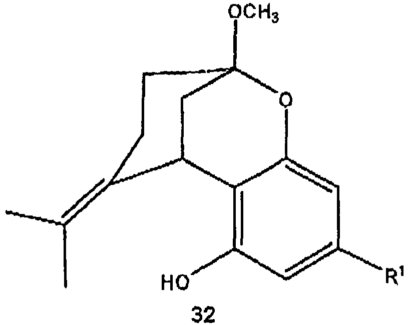

31 und 32 lassen sich wie bei Archer et al. beschrieben entweder direkt oder über den Umweg der cis-Verbindungen sauer katalysiert in die Verbindungen vom Typ trans-4 umlagern.

Die Ketofunktion kann dabei als Enolether, Enolester oder Ketal geschützt sein, wobei R³ und R⁴ geradkettige, verzweigte oder zyklische organische Gruppen mit bis zu 16 Kohlenstoffatomen oder siliciumorganische Reste mit bis zu 16 Kohlenstoffatomen sein können.

R³ und R⁴ können auch miteinander verbrückte geradkettige oder verzweigte Kohlenwas-serstoffreste sein mit bis zu 16 C-Atomen wie z. B. -(CH₂)ₙ-, - CH₂(CCH₃)₂CH₂-.

Die Gruppen R⁵ und R⁶ können dabei (Typ 10 bis15) Wasserstoff (H) oder eine alkoholische Schutzfunktion wie ein geradkettiges, verzweigtes oder zyklisches Al-kyl-, Acyl- oder ein siliciumorganischer Rest mit bis zu 16 C-Atomen sein.

Besondere Betonung verdient die Tatsache, dass im Falle der aufgeführten Verbindungen auch Ether und Ester (R⁵ bzw. R⁶ = geradkettiges , verzweigtes oder zyklisches Alkyl-, Acyl-, Siliciumorganyl- mit jeweils bis zu 16 C-Atomen) unter den Bedingungen der sauren Terpenylierung (Reaktionsschritt "a") wie die entsprechenden freien Alkohole (R⁵ bzw. R⁶ = H) reagieren können.

### Optisch aktive Enantiomeren von Nabilon und deren Homologe:

Werden in 4-Stellung optisch aktiv substituierte 12 oder 13 bzw. am C5 eindeutig sterisch definierte Verbindungen 14 oder 15 wie z. B. (1S,5S)-6,6-Dimethylbicyclo[3.1.1]hept-3-en-2-on = [(+)-Apoverbenon] = (1S.5S)-14: 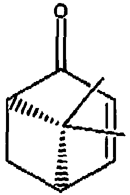 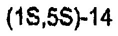 (1S,5S)-6,6-Dimethylbicyclo[3.1.1]hept-3-en-2,2-diyl diacetat = (1S,5S)-14a (R³ = R4 = CO-CH₃) [= (+)-6,6-Dimethyl-2,2-diacetoxy-3-norpinen]: 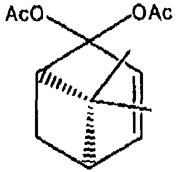 (1S,5S)-14a (R³ = R4 = CO-CH₃)
oder
(1S,5R)-6,6-Dimethylbicyclo[3.1.1]hept-2-en-2,4-diyl diacetat [= (-)-6,6-Dimethyl-2,4-diacetoxy-2-norpinen] = (1S,5S)-15b, (R³ = R⁵ = CO-CH₃) 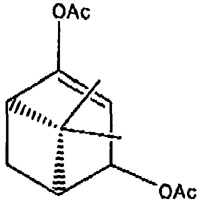 (1S,5S)-15b, (R³ = R⁵ = CO-CH₃)
mit Verbindungen vom Typ 5 kondensiert, so lassen sich unter Beibehalt der absoluten Konfiguration des C4 im Menthan-Gerüst bzw. am C5 des Bicyclo[3.3.1]heptens optisch aktive 9-Ketocannabinoide aufbauen.

(+)-Apoverbenon = (1S,5S)-14 kondensiert mit Verbindungen vom Typ 5 direkt zu den optisch aktiven trans-Estem 28-A und 28-B, welche nach decarboxylierender Verseifung b die entsprechenden esterfreien Verbindungen 33 ergeben: 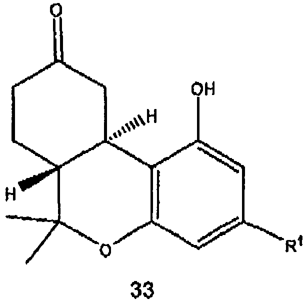 (1S,5S)-14a und (1S,5S)-15b kondensieren mit 5 zu Verbindungen vom Typ 34:

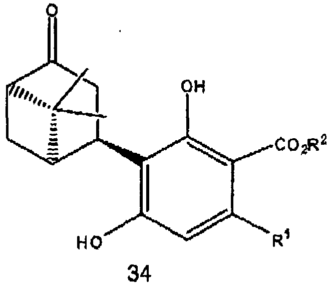

34 lassen sich sauer katalysiert (z. B. mit SnCl₄) in Verbindungen vom Typ 28-A und -B umlagern, aus welchen nach decarboxylierender Verseifung 33 entstehen.

Auch hier gilt, dass die decarboxylierende Verseifung (Schritt b) vor der sauer katalysierten Umlagerung stattfinden kann.

Im letzteren Fall wird aus 34 zuerst 35 hergestellt, welches dann sauer katalysiert zu 33 umgelagert wird.

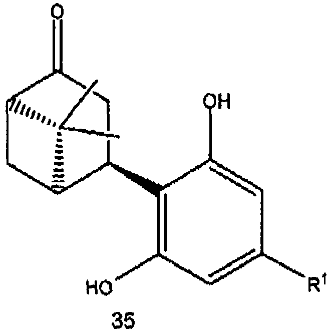

Ganz allgemein lässt sich für alle hier vorgestellten Produkte, bei denen eine sauer katalysierte Umlagerung Teil des Syntheseverfahrens ist, diese vor oder nach der decarboxylierenden Verseifung "b" durchführen.

Für den Kondensationsschritt (Schritt "a") geeignete Säuren sind sowohl Brönstedt-Säuren wie auch Lewis-Säuren:
Beispiele für geeignete Brönstedt-Säuren:
   Perchlorsäure, Halogenwasrstoffsäuren (HF, HCI, HBr, HI), Schwefelsäure, Hydrogensulfate, Phosphorsäure und ihre sauren Salze, Pyro- und Polyphosphorsäuren, organische Carbon- und Sulfonsäuren mit einem bis zu 30 Kohlenstoffatomen und einer oder mehre-ren sauren Gruppen, sowie an polymere Träger gebundene saure Gruppen wie z.B. saure lonenaustauscher und Mischungen der genannten Säuren. Namentlich genannt seien Ameisensäure, Oxalsäure, Trifluoressigsäure, p-Toluolsulfonsäure
Beispiele für geeignete Lewis-Säuren:
   Die Kationen von Erdalkali- und Erdmetallen sowie Übergangsmetallen; die Halogenverbindungen und andere dreiwertige Verbindungen von Elementen der dritten Hauptgruppe wie Bortrifluorid und andere Bor-Halogenverbindungen und ihre Komplexe, Aluminiumhalogenide wie wasserfreies Aluminiumchlorid; Salze und Halogenverbindungen von Übergangsmetallen wie Titantetrachlorid, Zinkchlorid, Zinktrifluormethansulfonat;

Halogenverbindungen von Elementen der vierten und fünften und sechsten Hauptgruppe wie z. B. Zinntetrachlorid, Phosphortrichlorid, Phosphorpentachlorid, Phosphoroxychlo-rid, Antimonpentafluorid, Thionylchlorid, Sulfurylchlorid, allein oder in Mischung mit anderen Lewis- oder Brönstedt-Säuren. An polymere Gerüste gebundene positive Zentren wie Montmorillonit,

Weitere geeignete Reagenzien zur Durchführung der Kondensation sind die Acetale von N,N-Dimethyfformamid wie z.B. N,N.Dimethylformamid-dineopentylacetal und andere wasserabspaltende Reagenzien, zum Beispiel solche, wie sie für die Bildung von Amiden und Peptiden Verwendung finden wie z. B. "T3P" (Propanphosphonsäureanhydrid),

Diese Reagenzien können als solche zur Reaktionsmischung dazugegeben werden oder auf ein Trägermaterial wie z.B. Aluminiumoxid aufgebracht sein.

Geeignete Lösungsmittel zur Durchführung des Kondensationsschrittes sind mit Wasser nicht mischbare oder mit Wasser mischbare Lösungsmittel wie z. B. Kohlenwasserstoffe mit bis zu 30 Kohlenstoffatomen, halogenierte Kohlenwasserstoffe mit bis zu 20 C-Atomen wie z.B. Dichlormethan oder Chloroform, Ether wie z. B. 2-Methoxytetrahydrofuran, Alkohole, Carbonsäuren mit bis zu 16 C-Atomen, Amide mit bis zu 20 C-Atomen, Ester mit bis zu 60 C-Atomen, Kohlendioxid. Schwefeldioxid, Wasser, Wasser mit einem Phasentransferkatalysator, die sauren Katalysatoren selbst, sowie Mischungen der genannten Lösungsmittel untereinander.

Die nämlichen Säuren und Lösungsmittel kommen auch für die erwähnten Isomerisierungs- und Epimerisierungsraktionen (Reaktionsschritt "c") zur Anwendung, man wählt dabei lediglich in der Regel etwas energischere Bedingungen wie z.B. höhere Temperaturen.

### Die Erfindung wird nun anhand der Beispiele näher erläutert.

Das Kondensationsverfahren ("a") und die beiden Methoden zur decarboxylierenden Verseifung ("b") seien im Folgenden an der Herstellung von Cannabidiol (CBD) aus p-Mentha-2,8-dien-1-ol und Olivetolsäuremethyloster (6-n-Pentyl-2,4-dihydroxybenzoesäuremethylester) als Beispiel erläutert.

Als Beispiel für eine Umlagerung (Isomerisierung, Reaktionsschritt "c") sei die Synthese von Dronabinol aus CBD aufgeführt

### Schritt 1: Kondensation von p-Mentha-2,8-dienol mit Olivetolsäuremethylester (Methode "a"):

Dieser Schritt ist identisch, egal ob danach mit dem Druckverfahren oder drucklos verseift und decarboxyliert wird oder ob eine nachfolgende Isomerisierung "c" vor oder nach der Verseifung "b" stattfindet

In einem 10-Liter Dreihalskolben mit Rührer, Rückflusskühler, Innenthermometer, Tropftrichter und Rückflusskühler werden vorgelegt:
- 300 g (1,259 mol) Olivetolsäuremethylester
- 196,4 g (1,290 mol) p-Mentha-2,8-dien-1-ol
- 2,5 Liter Dichlormethan (vorzugsweise unstabilisierte, frisch destillierte Ware)
- fakultativ kann ein Wasser bindendes Mittel wie z. B. 100 g wasserfreies Natriumsulfat oder 120 g Magnesiumsulfat zugegeben werden.

Die Mischung wird gerührt bis eine homogene Lösung entstanden ist

Der Kolben wird in ein externes Kühlbad aus Eis-Kochsalz eingetaucht und es wird weiter gerührt, bis eine Innentemperatur von minus 15°C erricht ist.

In den Tropftrichter wird eine Lösung von 59,5 g (0.419 mol) Bortrifluorid-diethyletherat in 500 ml unstabilisiertem, trockenem Dichlormethan gegeben.

Die Bortifluorid-etherat-Lösung wird unter kräftigem Rühren und Außenkühlung innerhalb ca. einer Stunde zur Reaktionsmischung getropft, wobei eine Innentemperatur von ca. minus 15°C eingehalten wird.

Die Reaktionslösung wird gelblich und trüb.

Nachdem die Gesamtmenge Bortrifluorid-etherat zugegeben ist, wird noch ca. 15 min. bei minus 15°C nachgerührt

Der Kolben wird aus dem Eisbad genommen.

Anschließend wird eine Lösung von 180 g (1,8 mol) Kaliumhydrogencarbonat in l deionisiertem Wasser innerhalb ca. 30 min unter kräftigem Rühren einlaufen gelassen, wobei gegen Ende Kohlendioxidentwicklung auftritt.

Es wird noch zwei Stunden nachgerührt, anschließend in einen Scheidetrichter überführt und die wässrige Phase (pH ca. 8) abgetrennt und verworfen

Die organische Phase wird mit zwei Portionen von jeweils 1 l deionisiertem Wasser gewaschen.

Die organische Phase wird abgetrennt und am Rotationsverdampfer einrotiert zuletzt wird die Badtemperatur auf 90°C gesteigert und der Druck auf 3 mbar vermindert, um Restlösungsmittel zu entfernen.

Ausbeute: 466 g (99%) roher Cannabidiolsäuremethylester (CBDAMe), der ca. 10-20% unverändertes Ausgangsmaterial (Olivetolsäuremethylester) enthält.

### Reinigung des rohen Cannabidiolsäuremethylester (CBDAMe):

466 g roher Cannabidiolsäuremethylester werden unter sachtem Erwärmen (40°C) in 2 l eines geeigneten, mit Wasser nicht mischbaren Lösungsmittels, wie z.B. Petrolether (pe) oder Methyl-tert.-butylether (MTBE) gelöst.

Die Lösung wird mit zwei Portionen von je 0,8 10,5 N Natriumhydroxid extrahiert,

Die wässrigen Phasen werden vereinigt und können angesäuert werden zur Wiedergewinnung nicht reagierten Olivetolsäuremethylesters.

Die organische Phase wird mit zwei Portionen von je 0,5 1 deionisiertem Wasser gewaschen, in denen jeweils 20 g Natriumsulfat gelöst sein können, um die Phasentrennung zu verbessern.

Die organische Phase abtrennen und eindampfen am Rotationsverdampfer; zuletzt wird die Badtemperatur auf 90°C gesteigert und der Druck auf 3 mbar vermindert, um Restlösungsmittel zu entfernen.

Ausbeute: 376 g (80% d. Th.) CBDAMe mit ca. 80% Reinheit.

### Schritt 2: Verseifung und Decarboxylierung der Cannabinoidcarbonsäureester (Methoden "b")

Zuerst sei für diesen Schritt das **Druckverfahren** am Beispiel des Cannabidiolcarbonsäuremethylesters beschrieben: (Analog TH 338)

In einem 21 Edelstahlautoklaven mit magnetischem Rührer und Innenthermometer werden vorgelegt:
- 74,5 g (0.20 mol) Cannabidiolsäuremethylester
- 120 ml deiionisiertes Wasser
- 25,0 g (0.18 mol) Kaliumcarbonat
- 180 ml Methanol

Der Autoklav wird mit Argon gespült, verschlossen und auf einer Heizplatte mit Magnetrührer erhitzt.

Nachdem eine Innentemperatur von 140 - 150°C erreicht ist, wird vier bis fünf Stunden bei dieser Temperatur gerührt.

Danach wird auf < 40°C abkühlen gelassen und entspannt.

Der Autoklaveninhalt wird mit 250ml Methanol in einen Rundkolben überführt und durch vorsichtige (CO₂-Entwicklung - Schäumen!) Zugabe einer Lösung von 23,2 g (0,36 Val) Citronensäure in 150 ml deionisiertem Wasser neutralisiert.

Die entstehende Emulsion wird am Rotationsverdampfer einrotiert (Wiedergewinnung von wässrigem Methanol) und der Rückstand, bestehend aus CBD, Kaliumsalzen der Citronen-säure und restlichem Wasser zwischen 200 ml deionisiertem Wasser und 300ml Petrolether (oder einem anderen mit Wasser nicht mischbaren Lösungsmittel) durch Rotieren im 40° warmen Wasserbad gelöst.

Phasen im Scheidetrichter trennen, wässrige Phase verwerfen und die organische Phase waschen mit zweimal je 100 ml 3% iger Natriumsulfatlösung.

Nach Einrotieren der organischen Phase verbleiben 62,3 g (99% d. Th.) rohes CBD.

Im Folgenden ist das **drucklose Verfahren** zur Verseifung und Decarboxylierung der Ester-zwischenstufen anhand von CBDAMe beschrieben (Nach TH 502):
Die Apparatur besteht aus einem 10 l Dreihalskolben in einer Heizhaube mit Rührer, Innen-thermometer und einem Claisen-Ausatz mit 30 cm Vigreux-Kolonne und Gaseinleitungsrohr. Auf die Vigreux-Kolonne ist ein Destillationsaufsatz mit Kopfthermometer und absteigender Destillationsbrücke montiert, welche einen graduierten Kolben als Vorlage hat.

In den 10 l Kolben werden nacheinander gegeben:
- 540,1 g (1,45 mol) CBDAMe
- 2,0 l Monoethylenglykol
- eine Lösung von 67,4 g (1,08 mol) ca. 90%iger Kaliumhydroxidschuppen in
- 340 ml deionisiertes Wasser
- 0,5 g Edelstahlpulver

Die Apparatur wird 5 min mit ca. 5 l Argon/min gespült, dann wird begonnen unter Rühren zu erhitzen und der Argonstrom auf ca. 0,1 l/min reduziert.

Bei einer Innentemperatur von ca. 128°C beginnt der Kolbeninhalt zu sieden und bald darauf beginnt ein methanolreiches Gemisch über den Kolonnenkopf zu destillieren.

Es wird unter Rühren und Inertgaseinleiten vorsichtig weiter erhitzt, so dass kontinuierlich langsam Destillat übergeht.

Nach ca. 3 h Kochen haben sich ca. 140 ml Destillat gebildet, die Sumpftemperatur ist auf 140°C und die Kopftemperatur auf 100°C gestiegen.

Abkühlen lassen unter Inertgaseinleiten; dabei die Vigreux-Kolonne gegen einen Tropftrichter mit Druckausgleich austauschen.

Bei 85°C Innentemperatur 3 l deionisiertes Wasser zugeben und anschließend innerhalb ca, einer halben Stunde eine Lösung von 76,5 g (0,40 mol) Citronensäure in 1 l deionisiertem Wasser zutropfen (Schäumen gegen Ende des Zutropfens durch CO2-Entwicklung!).

Nun wird der Inertgasstrom gestoppt und bei einer Inneritemperstur < 40°C innerhalb einer halben Stunde 1,5 l Petrolether zutropfen (oder ein anderes mit Wasser nicht mischbares Lösungsmittel), wobei weiteres Kohlendioxid entweicht.

Mindestens 1 h hochtourig nachrühren.

Den Kolbeninhalt in einen Scheidetrichter überführen, die Unterphase abtrennen und mit 1 l Petrolether (oder einem anderen mit Wasser nicht mischbaren Lösungsmittel) nachextrahieren.

Die organischen Phasen vereinen und 5 mal mit 0,60 l deionisiertem Wasser waschen, wobei emulsionsartige Zwischenphase durch Zugabe von ca. 0,10 l 10%iger Natriumsulfatlösung gebrochen werden kann.

Die organische Phase abtrennen, einrotieren. Restlösungsmittel im Vakuum bei einer Badtemperatur von 90°C abziehen.

Ausbeute: 455.1 g (100% d. Th.) rohes CBD

Sowohl für das Druckverfahren wie auch für das drucklose Verfahren können die gleichen Basen zur Verseifung Verwendung finden.

### Verkürzung der Reaktionszeit durch Katalysatoren:

Sowohl für das drucklose wie auch das Druckverfahren lassen sich die Reaktionszeiten durch Zugabe eines geeigneten Katalysators wie z. B. 0,1 Gew. - % (bezogen auf CBDAMe) Edeistehlpulver oder 0,01 Gew. - % Silberpulver abkürzen. Dieser Katalysator beschleunigt die Decarboxylierung der als Zwischenprodukt entstehenden Carbonsäure.

Die weitere Reinigung des rohen Produkts erfolgt nach einem oder mehreren der folgenden Verfahren:
1. Destillation, 2. Kieselgelfiltration (Chromatographie), 3. Kristallisation und Umkristallisation.

Diese Verfahren können einzeln oder in beliebiger Kombination angewandt werden, um reines CBD zu gewinnen.

### 1. Destillation

Die Vakuumdestillation von CBD kann sowohl aus einem Sumpfkolben heraus wie auch aus einer Dünnschichtapparatur heraus erfolgen. Zweckmäßig destilliert man bei Drücken unter 1 mbar, vorzugsweise <0,3 mbar. Die Kühlflüssigkeit des Kondensators sollte ausreichend warm (> 50°C) sein, um eine ausreichende Abfließgeschwindigkeit des kondensierten CBDs zu gewährleisten:

### Fraktionierende Destillation von CBD aus einem Sumpfkolben:

### Apparatur:

1 l Rundkolben mit Heizhaube, Rührer, Sumpfthermometer und aufgesetzter Destillationsbrücke mit Kopfthermometer, Wechselvorlage zum Fraktionensammeln.

Thermostatisierbares Wasserbad mit Umwälzpumpe als Kühlflüssigkeit für die Destillationsbrücke.

Vakuumpumpe mit Druckmessgerät und mit flüssigem Stickstoff beschickter, vorgeschalteter Kühlfalle.

### Durchführung:

Der Destillationskolben wird mit 242 g rohem CBD beschickt.

Das rohe CBD wird auf ca. 60°C vorgewärmt und der Rührer gestartet.

Vorsichtig wird nun Vakuum angelegt und langsam die Sumpftemperatur gesteigert.

Mit einer Kühlflüssigkeitstemperatur von < 30°C destillieren bei einer Kopftemperatur von 50-60°C und einem Druck zwischen 3 mbar und 0,8 mbar 5 Gramm Vorlauf, der hauptsächlich aus Terpenen besteht.

Eine zweite Fraktion (16,4 g) mit 68% CBD destilliert bei einer Kopftemperatur von 120-132°C und einem Druck von 0,70 bis 0,14 mbar. Kühlwasser 60°C.

Die dritte Fraktion (178 g) besteht aus 90%igem CBD und destilliert bei 133-155°C Kopftemperatur und einem Druck von 0,10 bis 0,15 mbar. Kühlwasser 70°C.

Im Sumpfkolben bleiben 42 g Rückstand mit weniger als 5% CBD.

### Kurzweg-Dünnschichtdestillation von CBD:

Der Tropftrichter einer Kurzweg-Destillationsapparatur wie der KD 1 von UIC wird portionsweise beschickt mit 1971,4 g vorgewärmten (ca. 60°C) CBD.

. Der Heizmantel der Apparatur wird auf 180°Cgehalten.

Die Kühlfalle zur Vakuumpumpe (Öl-Drehschieberpumpe) wird mit Trockeneis-Aceton oder mit flüssigem Stickstoff beschickt.

Die Kühlflüssigkeit wird auf 60°C vorgewärmt

Bei 500 U/min wird nun innerhalb von ca. 12 h das CBD in die Apparatur eintropfen gelassen.

Bei einem Vakuum von 0,02 bis 0,3 mbar sammeln sich 1760 g Destillat und 178,2 g Destillationsrückstand in den jeweiligen Vorlagen, sowie 12,1 g Kondensat in der Kühlfalle.

### 2. Kieselgelfiltration (Chromatographie)

Kieselgel oder andere chromatographische Adsorbentien wie z. B. Aluminiumoxid können viele die Kristallisation von CBD behindernde Verunreinigungen zurückhalten, wenn adsorbiertes Roh-CBD mit einem geeigneten Lösungsmittel eluiert wird.

Geeignete Lösungsmittel sind Kohlenwasserstoffe, halogenierte Kohlenwasserstoffe, Ester, Ether und Ketone mit bis zu 20 Kohlenstoffatomen, sowie deren Gemische untereinander.

Zur Durchführung löst man ein Gewichtsteil des zu reinigenden CBDs in einem geeigneten ersten Lösungsmittel wie n-Heptan, und gibt diese Lösung auf ein Kieselgelbett aus einem, vorzugsweise zwei Gewichtsteilen Kieselgel zur Chromatographie.

Man lässt das erste Lösungsmittel ablaufen und eluiert (wäscht) nun das Kieselgelbett mit einem geeignetem Lösungsmittel oder Gemisch wie z. B. einem Volumenteil Dichlormethan und 4 Volumenteilen Heptan bis kein CBD mehr im Eluat nachweisbar ist.

Durch Eindampfen des Eluats wird das gereinigte CBD gewonnen; die zurückgehaltenen Verunreinigungen können mit dem verbrauchten Kieselgel entsorgt werden.

Durch Sammeln von Fraktionen lassen sich unterschiedlich reine Qualitäten von CBD darstellen und die Verunreinigungen getrennt vom CBD eluieren.

### Kristallisation und Umkristallisation:

Rohes CBD, vorzugsweise durch Destillation oder Kieselgelfiltration vorgereinigtes CBD, lässt sich durch Lösen in einem geeignetem Lösungsmittel, Abkühlen der Lösung und Animpfen zur Kristallisation bringen.

Dieses Reinigungsverfahren ist bei entsprechender Durchführung verlustarm und liefert hervorragende Reinigungswirkung.

Geeignete Lösungsmittel zur Kristallisation sind Kohlenwasserstoffe mit drei bis 30 Kohlenstoffatome, vorzugsweise geradkettige Kohlenwasserstoffe wie n-Pentan, n-Hexan, n-Heptan.

Ferner geeignet sind hochfluorierte oder teilfluorierte kettenförmige Kohlenwasserstoffe und Ester von gesättigten oder ungesättigten kettenförmigen Carbonsäuren mit einem bis zu 36 Kohlenstoffatomen mit kettenförmigen Mono-, Di- oder Oligoalkoholen wie z.B. Glycerin, sowie Mischungen der genannten Lösungsmittel.

### Beispiel:

1755,5 g destilliertes Roh-CBD (amorph) werden unter Erwärmen und Rühren in 7,6 1 n-Pentan gelöst.

Die Lösung wird unter ständigem Rühren abgekühlt und wiederholt angeimpft. Bei einer Temperatur < 20°C beginnt das CBD zu kristallisieren.

Unter weiterem Rühren wird abgekühlt auf minus 38°C und der entstehende Kristallbrei von CBD in der Kälte abgesaugt und nachgewaschen mit 1,5 l kaltem n-Pentan.

Nach Trocknen verbleiben 1313,3 g kristallines CBD.

### Umkristallisation:

1010,1 g kristallines CBD werden analog in 3,8 In-Pentan warm gelöst. Unter Rühren und Animpfen abkühlen auf minus 38°C.

Absaugen und nachwaschen mit 1,5 l eiskaltem (< minus 30°C) n-Pentan.

Ausbeute nach Trocknen 985,7g.

### Schritt 3: Sauer katalysierte Isomerisierung (Reaktion "c")

Falls eine sauer katalysierte Umlagerung (Isomerisierung/Epimerisierung) zum erwünschten Endprodukt führt, kann diese prinzipiell vor oder nach der decarboxylierenden Verseifung (Reaktion "b") durchgeführt werden.

Grundsätzlich finden die gleichen Säuren und Lösungsmittel Anwendung, wie im Kondensationsschritt "a".

Durch die Wahl der Säure, des Lösungsmittels und der entsprechenden Temperatur lässt sich die Reaktion in gewünschter Weise steuern.

Die oben erwähnten Isomerisierungen (Ringschlussreaktionen, Epimerisierungen und Umlagerungen am Kohlenstoffskelett) seien hier am Beispiel der Synthese von Dronabinol aus Cannabidiol erläutert.

### Beispiel für eine sauer katalysierte Isomerisierung (Schritt "c"):

In einem 2 l Dreihalskolben mit Rührer, Tropftrichter und Trockenrohr werden 31 g Cannabidiol in 1,01 Dichlormethan gelöst.

Optional kann ein alkalisches Trockenmittel wie Kaliumcarbonat oder basisches Aluminimoxid zugegeben werden.

Nun wird unter Rühren eine Lösung von 5,0 g Bortrifluorid-etherat in 100ml Dichlormethan eingetropft.

Es wird bei Raumtemperatur gerührt und in Abständen von 15 min der Reaktionsfortschritt mit Hilfe von Gaschromatographie überprüft.

Gegen Reaktionsende steigt der Gehalt an delta-8- Tetrahydrocannabinol überproportional an.

Wenn der Gehalt an delta-8-Tetrahydrocannabinol 2% relativ zu delta-9-eeTetrahydro-cannabinol beträgt, wird die Reaktion abgebrochen durch Zugabe von 300 ml 5%iger Natriumhydrogencarbonatlösung.

Man rührt eine Stunde nach, trennt die Phasen, wäscht die organische Phase nacheinander mit 300 ml 5%iger Natriumhydrogencarbonatlösung und zweimal mit je 300 ml deionisiertem Wasser.

Anschließend wird die organische Phase eingeengt und der Rückstand chromatographisch an Kieselgel gereinigt.

Ausbeute: 27,9 g (90% d. Th.) reines Dronabinol.

## Patentansprüche

1. Verfahren zur Herstellung einer Verbindung der allgemeinen Formel Ia, Ib und deren Diastereoisomere, 22 und 35 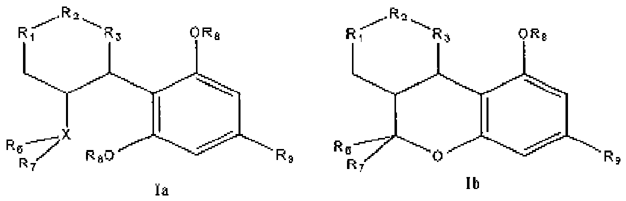 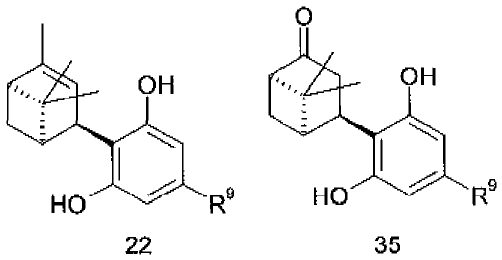 durch decarboxylierende Verseifung der Verbindungen der allgemeinen Formel IIa IIb. IIc bzw.21 oder 34 unter Druck in einem niedrig siedenden Lösungsmittel 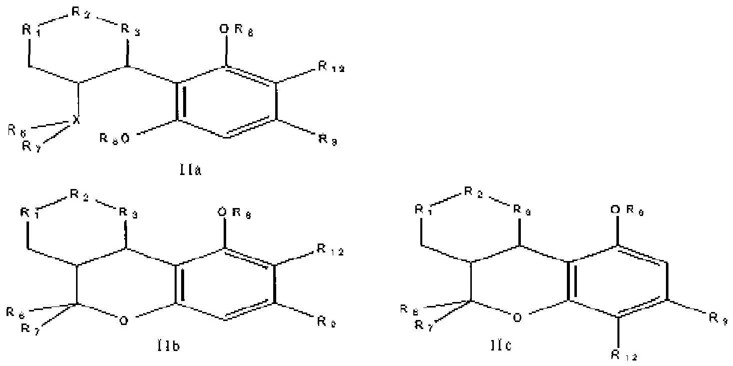 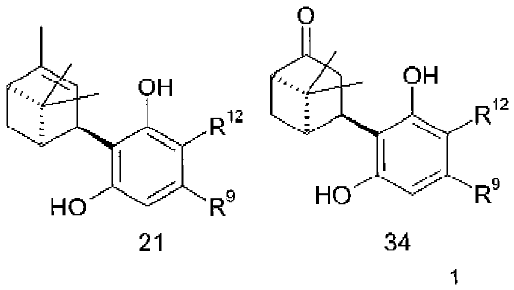 wobei
eine der Bindungen R₁-R₂ oder R₂-R₃ eine C-C Doppelbindung sein kann, und
R₁ und R3 CH₂ oder CH sind, und
R₂ entweder C=O oder eine R10-C-R11 Gruppe ist,
wobei
R₁₀ und R11 unabhängig voneinander entweder H oder eine niedere C₁-C₄ Alkylgruppe sind, wenn keine Doppelbindung zwischen R1 und R3 vorhanden ist, oder falls eine Doppelbindung zwischen R1 und R3 vorhanden ist, eine der Gruppen R10 oder R₁₁ entfällt, und die andere wie oben definiert ist;
X entweder C ist, wenn R₆ eine =CH₂ Gruppe ist und R7 eine CH₃ Gruppe ist, oder X eine CR4 Gruppe ist, wobei R4 H oder eine niedere Alkylgruppe ist, CH oder eine C-O-R₅ Gruppe ist und R5 entweder H, eine C₁ - C₁₆ Alkylgruppe oder eine Schutzgruppe ist;
R6 und R7 eine CH₃ Gruppe sind oder wenigstens eine der Gruppen R₆ und R7 eine CH2= Gruppe darstellt und die andere eine CH₃ Gruppe ist,
R₈ eine C₁ - C₁₆ Alkylgruppe, H oder eine Schutzgruppe ist,
R9 eine C1-C16-, oder O-C₁-C₁₆-Gruppe ist, wobei C₁-C₁₆ eine gerade oder verzweigte Alkylkette darstellt, die an beliebiger Stelle eine oder mehrere Doppel- oder Dreifachbindungen aufweist oder Substituenten wie Deuterium- oder Halogen-Atome, Phenyl-, substituierte Phenyl-, Cycloalkyl-, Nitril-, Alkoxy-Gruppen oder eine Ketogruppe aufweisen kann,
R₁₂ eine CO₂-R₁₃-Gruppe ist, und
R₁₃ eine C₁-C₁₆-Alkylgruppe oder eine Schutzgruppe ist,
wobei die decarboxylierende Verseifung im alkalischen Medium durchgeführt wird.

2. Verfahren zur Herstellung einer Verbindung der allgemeinen Formel Ia, Ib und deren Diastereoisomere, 22 oder 35 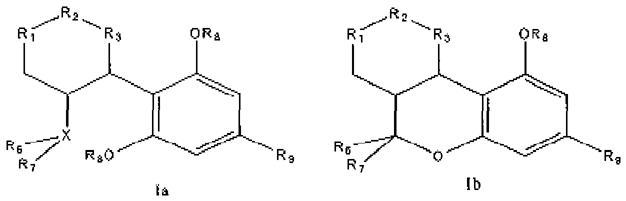 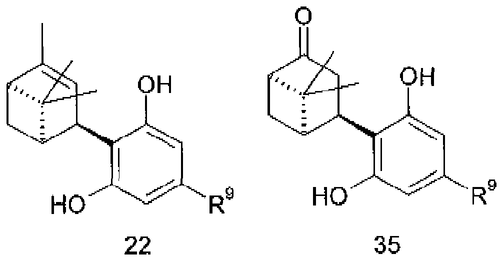 durch decarboxylierende Verseifung der Verbindungen der allgemeinen Farmel IIa IIb, IIc bzw 21 oder 34 bei atmosphärischem Druck in einem über 100°C siedenden Lösungsmittel 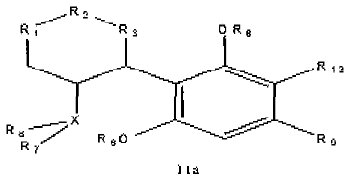 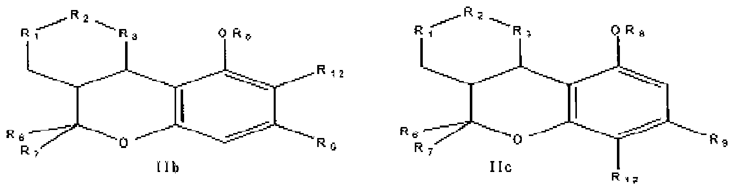 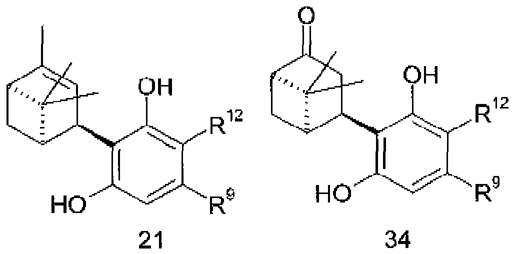 wobei
eine der Bindungen R₁-R₂ oder R₂-R₃ eine C-C Doppelbindung sein kann, und
R₁ und R₃ CH₂ oder CH sind, und
R₂ entweder C=O oder eine R₁₀-C-R₁₁ Gruppe ist,
wobei
R₁₁ und R₁₁ unabhängig voneinander entweder H oder eine niedere C₁-C₄ Alkylgruppe sind, wenn keine Doppelbindung zwischen R₁ und R₃ vorhanden ist, oder falls eine Doppelbindung zwischen R₁ und R₃ vorhanden ist, eine der Gruppen R₁₀ oder R₁₁ entfällt, und die andere wie oben definiert ist;
X entweder C ist, wenn R₆ eine =CH₂ Gruppe ist und R₇ eine CH₃ Gruppe ist, oder
X eine CR₄ Gruppe ist, wobei R₄ H oder eine niedere Alkylgruppe ist, CH oder eine C-O-R₅ Gruppe ist und R₅ entweder H, eine C₁ - C₁₆Alkylgruppe oder eine Schutzgruppe ist;
R₆ und R₇ eine CH₃ Gruppe sind oder wenigstens eine der Gruppen Rs und R₇ eine CH₂= Gruppe darstellt und die andere eine CH₃ Gruppe ist,
R₈ eine C₁ - C₁₆ Alkylgruppe, H oder eine Schutzgruppe ist,
R₉ eine C₁-C₁₆-, oder O-C₁-C₁₆-Gruppe ist, wobei C₁-C₁₆ eine gerade oder verzweigte Alkylkette darstellt, die an beliebiger Stelle eine oder mehrere Doppel- oder Dreifachbindungen aufweist oder Substituenten wie Deuterium- oder Halogen-Atome, Phenyl-, substituierte Phenyl-, Cycloalkyl-, Nitril-, Alkoxy-Gruppen oder eine Ketogruppe aufweisen kann,
R₁₂ eine CO₂-R₁₃-Gruppe ist, und
R₁₃ eine C₁-C₁₆-Alkylgruppe oder eine Schutzgruppe ist,
wobei die decarboxylierende Verseifung im alkalischen Medium durchgeführt wird.

3. Verfahren nach einem der Ansprüche 1 oder 2, **dadurch gekennzeichnet, dass** Decarboxylierungskatalysatoren, beispielsweise Übergangsmetalle und deren Salze, vorzugsweise Edelstahlpulver oder Silberpulver verwendet werden.

4. Verfahren nach Anspruch 1, **dadurch gekennzeichnet, dass** als Lösungsmittel niedere Alkohole mit bis zu fünf Kohlenstoff-Atomen, Ammoniak, sowie deren Mischungen untereinander und mit Wasser verwendet werden.

5. Verfahren nach Anspruch 1, **dadurch gekennzeichnet, dass** das Verfahren im wässrigen Medium mit Phasentransferkatalysatoren oder Emulgatoren durchgeführt wird.

6. Verfahren nach Anspruch 2, **dadurch gekennzeichnet, dass** mindestens eines der folgenden Lösungsmittel DMF, DMSO, Sulfolan, Furfurol, Di- und Tetrahydrofurfurol, 2-Methoxytetrahydrofuran, Hexamethylenphosphorsäuretriamid, Acetamid, Amide mit bis zu 12 C-Atomen, Tetramethyl-Harnstoff, Ethylenglykol, sowie seine Mono- und Bis-Ether, Ethanolamin, Ethylendiamin, Propylenglykol und seine Ether mit bis zu 20 C-Atomen, Glycerin und Glycerin-Ether mit bis zu 30 C-Atomen, 1,2-butandiol, 1,3-Butandiol, 1,4-Butandiol, Diethylenglykol und seine Ether, Triethylenglykol und seine Ether, Polyethylenglykol, Polyvinylpyrrolidon mit oder ohne Wasser verwendet werden.

7. Verfahren nach einem der Ansprüche 1 bis 6, **dadurch gekennzeichnet, dass** zur alkalischen Verseifung eine oder mehrere der folgenden Basen verwendet werden: Hydroxide von Alkali- und Erdalkalimetallen sowie von quarternären AmmoniumSalzen, Carbonate, Hydrogencarbonate und Carboxylate mit bis zu 30 C-Atomen, Phenolate, Phosphate, Phosphite, Sulfide, Hydrogensulfide, Mercaptide mit bis zu 30 C-Atomen, Sulfite, Hydrogensulfite, Cyanide von Alkalimetallen und quaternären Ammonium-Salzen mit 4 bis 48 C-Atomen, Ammoniak, organische Alkyl-, Aryl- oder aromatische oder nicht aromatische heterocyclische Amine mit bis zu 36 C-Atomen sowie deren Salze, Borate, Tetraborate.

8. Verfahren nach einem der Ansprüche 1 oder 2, **dadurch gekennzeichnet, dass** das Esterzwischenprodukt der Verbindung der Formel Ia, Ib, 22 oder 35 durch eine säurekatalysierte Kondensation mit einer geeigneten ungesättigten Terpenvorstufe mit der Verbindung der allgemeinen Formel III hergestellt wird, wobei R₈, R₉ und R₁₂ wie in Anspruch 1 definiert sind.

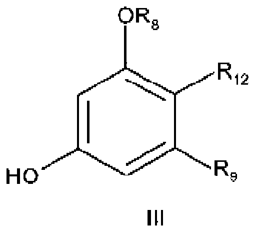

9. Verfahren nach Anspruch 8, **dadurch gekennzeichnet, dass** die Kondensation in Anwesenheit von Acetalen von N,N-Dimethylformamid wie z.B. N,N-Dimethylformamid-dineopentylacetal oder anderen wasserabspaltenden Reagenzien, vorzugsweise Propanphosphonsäureanhydriddurchgeführt wird, wobei diese als solche zu der Reaktionslösung gegeben werden oder auf ein Trägermaterial wie z.B. Aluminiumoxid aufgebracht sein können, in Anwesenheit von mindestens einer der folgenden Brönstedt- oder Lewis-Säuren durchgeführt wird: Perchlorsäure, Halogenwasserstoffsäuren (HF, HCl, HBr, HI), Schwefelsäure, Hydrogensulfate, Phosphorsäure und ihre sauren Salze, Pyro- und Polyphosphorsäuren, organische Carbon- und Sulfonsäuren mit einem bis zu 30 Kohlenstoffatomen und einer oder mehreren sauren Gruppen, sowie an polymere Träger gebundene saure Gruppen wie z.B. saure Ionenaustauscher und Mischungen der genannten Säuren, bspw. Ameisensäure, Oxalsäure, Trifluoressigsäure, p-Toluolsulfonsäure. Kationen von Erdalkali- und Erdmetallen sowie Übergangsmetallen; Halogenverbindungen und andere dreiwertige Verbindungen von Elementen der dritten Hauptgruppe wie Bortrifluorid und andere Bor-Halogenverbindungen und ihre Komplexe, Aluminiumhalogenide wie wasserfreies Aluminiumchlorid; Salze und Halogenverbindungen von Übergangsmetallen wie Titantetrachlorid, Zinkchlorid, Zinktrifluormethansulfonat; Halogenverbindungen von Elementen der vierten und fünften und sechsten Hauptgruppe, z. B. Zinntetrachlorid, Phosphortrichlorid, Phosphorpentachlorid, Phosphoroxychlorid, Antimonpentafluorid, Thionylchlorid, Sulfurylchlorid, entweder allein oder in Mischungen mit anderen Brönstedt- oder Lewis-Säuren; an polymere Gerüste gebundene positive Zentren wie bspw. Montmorillonit.

10. Verfahren nach Anspruch 8 oder 9, **dadurch gekennzeichnet, dass** die Kondensation in mindestens einem der folgenden mit Wasser mischbaren oder mit Wasser nicht mischbaren Lösungsmittel durchgeführt wird: Kohlenwasserstoffe mit bis zu 30 C-Atomen, halogenierte Kohlenwasserstoffe mit bis zu 20 C-Atomen wie z.B. Dichlormethan oder Chloroform, Ether wie z. B. 2-Methoxytetrahydrofuran, Alkohole, Carbonsäuren mit bis zu 16 C-Atomen, Amide mit bis zu 20 C-Atomen, Ester mit bis zu 60 C-Atomen, Kohlendioxid, Schwefeldioxid, Wasser, Wasser mit einem Phasentransferkatalysator, die sauren Katalysatoren selbst, sowie Mischungen der genannten Lösungsmittel untereinander.

11. Verfahren nach einem der Ansprüche 1 oder 2, **dadurch gekennzeichnet, dass** die Verbindung der Farmel 1a: 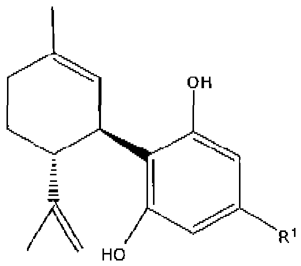 1a mit R¹ = n-C₅H₁₁
durch decarboxylierende Verseifung der Verbindung der Formel 16a 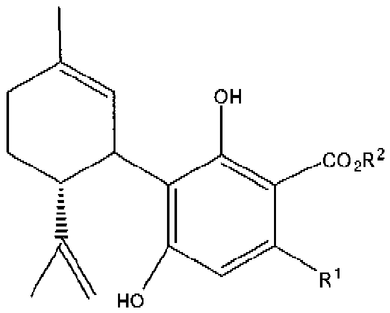 16a mit R¹ = n-C₅H₁₁; R² = CH₃
hergestellt wird.

12. Verfahren nach einem der Ansprüche 8 bis 11, **dadurch gekennzeichnet, dass** die Verbindung der Formel 16a 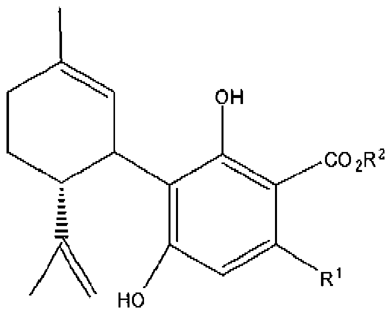 16a mit R¹ = n-C₅H₁₁; R² = CH₃
durch eine säurekatalysierte Kondensation der Verbindungen der Formeln 6a und 5a 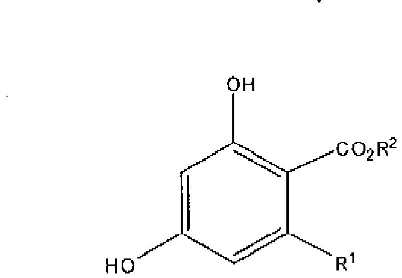 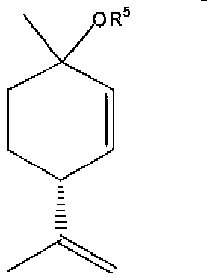 5a mit R¹ = n-C₅H₁₁; R² = CH₃ 6a mit R⁵ = H
hergestellt wird.

13. Verfahren nach Anspruch 11, **dadurch gekennzeichnet, dass** aus der Verbindung der Formel 1a in einem nachfolgenden sauer katalysierten Isomerisierungsschritt (-)-delta-9- Tetrahydrocannabinol (Dronabinol, Formel 2a) 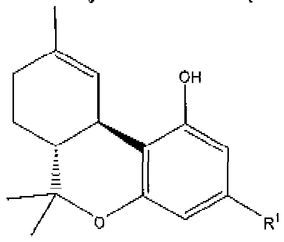 2a mit R¹ = n-C₅H₁₁
hergestellt wird.

14. Verfahren nach einem der vorstehenden Ansprüche, **dadurch gekennzeichnet, dass** vor oder nach der decarboxylierenden Verseifung eine säurekatalysierte Umlagerung durchgeführt wird.

15. Verfahren nach Anspruch 14, **dadurch gekennzeichnet, dass** die Umlagerung eine Epimerisierung ist

16. Verfahren zur Herstellung von Verbindungen der allgemeinen Formel Ia, Ib, 22 oder 35 gemäß Anspruch 1 oder 2, **gekennzeichnet durch** folgende Schritte:
a) Umsetzen von Verbindungen der allgemeinen Formel III mit einem geeignetem ungesättigtem Terpen gemäß Anspruch 8, um eine Verbindung der allgemeinen Formel IIa, IIb, IIc, 21 oder 34 gemäß Anspruch 1 oder 2 zu erhalten;
b) Durchführen einer decarboxylierenden Verseifung gemäß Anspruch 1 oder 2; und
c) Durchführen einer säurekatalysierten Umlagerung,
wobei der Schritt c) vor dem Schritt b) durchgeführt werden kann.

## Claims

1. Process for preparing a compound of the general formula Ia, Ib and the diastereoisomers 22 and 35 thereof 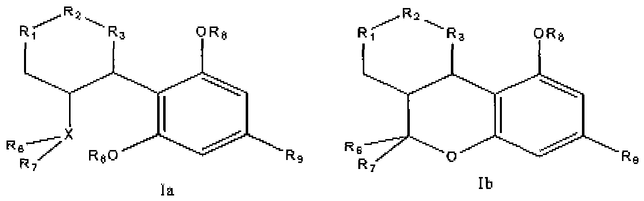 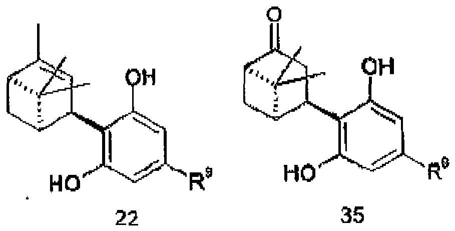 by decarboxylating hydrolysis of the compounds of the general formula IIa, IIb, IIc or 21 or 34 under pressure in a low-boiling solvent 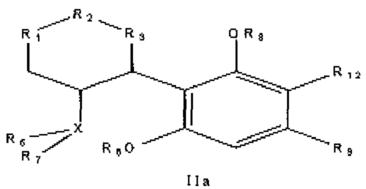 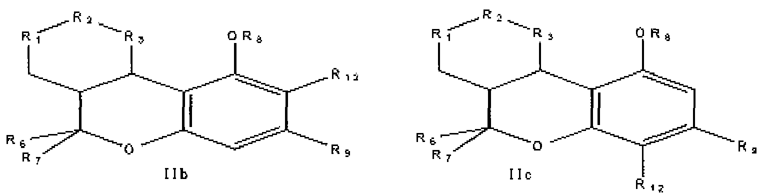 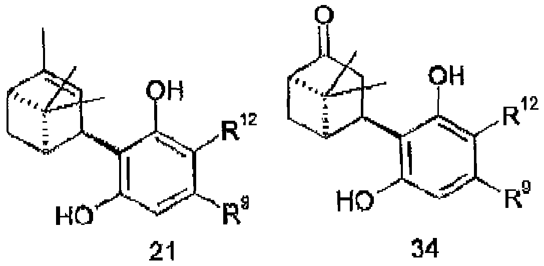 where
one of the bonds R₁-R₂ or R₂-R₃ may be a C-C double bond, and
R₁ and R₃ are each CH₂ or CH, and
R₂ is either C=O or an R₁₀-C-R₁₁ group,
where
R₁₀ and R₁₁ are each independently H or a lower C₁-C₄ alkyl group when a double bond is not present between R₁ and R₃, or, if a double bond is present between R₁ and R₃, one of the R₁₀ and R₁₁ groups is absent and the other is as defined above;
X is either C when R₆ is a =CH₂ group and R₇ is a CH₃ group, or
X is a CR₄ group where R₄ is H or a lower alkyl group, CH or a C-O-R₅ group, and R₅ is H, a C₁ - C₁₆ alkyl group or a protecting group;
R₆ and R₇ are each a CH₃ group or at least one of the R₆ and R₇ groups is a CH₂= group and the other is a CH₃ group,
R₈ is a C₁ - C₁₆ alkyl group, H or a protecting group,
R₉ is a C₁-C₁₆ or O-C₁-C₁₆ group, where C₁-C₁₆ is a straight or branched alkyl chain which has one or more double or triple bonds at any position or may have substituents such as deuterium or halogen atoms, phenyl, substituted phenol, cycloalkyl, nitrile, alkoxy groups or a keto group,
R₁₂ is a CO₂-R₁₃ group, and
R₁₃ is a C₁-C₁₆ alkyl group or a protecting group, wherein the decarboxylating hydrolysis is performed in an alkaline medium.

2. Process for preparing a compound of the general formula Ia, Ib and the diastereoisomers 22 and 35 thereof 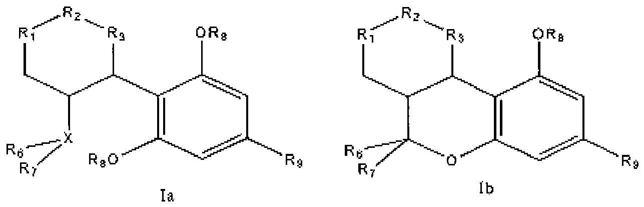 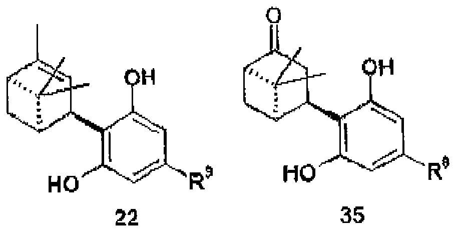 by decarboxylating hydrolysis of the compounds of the general formula IIa, IIb, IIc or 21 or 34 at atmospheric pressure in a solvent which boils above 100°C 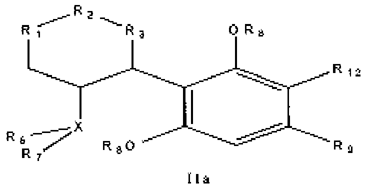 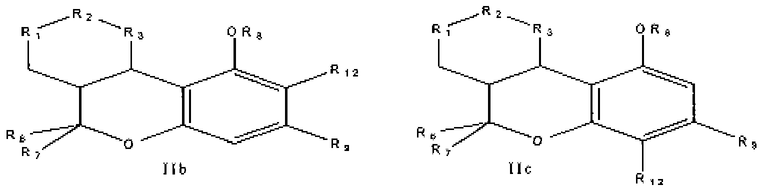 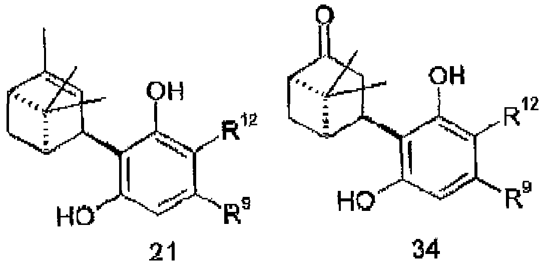 where
one of the bonds R₁-R₂ or R₂-R₃ may be a C-C double bond, and
R₁ and R₃ are each CH₂ or CH, and
R₂ is either C=O or an R₁₀-C-R₁₁ group,
where
R₁₀ and R₁₁ are each independently H or a lower C₁-C₄ alkyl group when a double bond is not present between R₁ and R₃, or, if a double bond is present between R₁ and R₃, one of the R₁₀ and R₁₁ groups is absent and the other is as defined above;
X is either C when R₆ is a =CH₂ group and R₇ is a CH₃ group, or
X is a CR₄ group where R₄ is H or a lower alkyl group, CH or a C-O-R₅ group, and R₅ is H, a C₁ - C₁₆ alkyl group or a protecting group;
R₆ and R₇ are each a CH₃ group or at least one of the R₆ and R₇ groups is a CH₂= group and the other is a CH₃ group,
R₈ is a C₁ - C₁₆ alkyl group, H or a protecting group,
R₉ is a C₁-C₁₆ or O-C₁-C₁₆ group, where C₁-C₁₆ is a straight or branched alkyl chain which has one or more double or triple bonds at any position or may have substituents such as deuterium or halogen atoms, phenyl, substituted phenyl, cycloalkyl, nitrile, alkoxy groups or a keto group,
R₁₂ is a CO₂-R₁₃ group, and
R₁₃ is a C₁-C₁₆ alkyl group or a protecting group, wherein the decarboxylating hydrolysis is performed in an alkaline medium.

3. Process according to either of Claims 1 and 2, **characterized in that** decarboxylation catalysts, for example transition metals and salts thereof, preferable stainless steel powder or silver powder, are used.

4. Process according to Claim 1, **characterized in that** solvents used are lower alcohols having up to five carbon atoms, ammonia, and mixtures thereof with one another and with water.

5. Process according to Claim 1, **characterized in that** the process is performed in aqueous medium with phrase transfer catalysts or emulsifiers.

6. Process according to Claim 2, **characterized in that** at least one of the following solvents is used: DMF, DMSO, sulpholane, furfurol, di- and tetrahydrofurfurol, 2-methoxytetrahydrofuran, hexamethylenephosphoramide, acetamide, amides having up to 12 carbon atoms, tetramethylurea, ethylene glycol, and the mono- and bisethers thereof, ethanolamine, ethylenediamine, propylene glycol and ethers thereof having up to 20 carbon atoms, glycerol and glyceryl ethers having up to 30 carbon atoms, 1,2-butanediol, 1,3-butanediol, 1,4-butanediol, diethylene glycol and ethers thereof, triethylene glycol and ethers thereof, polyethylene glycol, polyvinylpyrrolidone with or without water.

7. Process according to any of Claims 1 to 6, **characterized in that** one or more of the following bases are used for alkaline hydrolysis:
hydroxides of alkali metals and alkaline earth metals and of quaternary ammonium salts, carbonates, hydrogencarbonates and carboxylates having up to 30 carbon atoms, phenoxides, phosphates, phosphites, sulphides, hydrogensulphides, mercaptides having up to 30 carbon atoms, sulphites, hydrogensulphites, cyanides of alkali metals and quaternary ammonium salts having 4 to 48 carbon atoms, ammonia, organic alkylamines, arylamines or aromatic or nonaromatic heterocyclic amines having up to 36 carbon atoms, and the salts thereof, borates, tetraborates.

8. Process according to either of Claims 1 and 2, **characterized in that** the ester intermediate of the compound of the formula Ia, Ib, 22 or 35 is prepared by an acid-catalysed condensation of a suitable unsaturated terpene precursor with the compound of the general formula III where R₈, R₉ and R₁₂ are each as defined in Claim 1

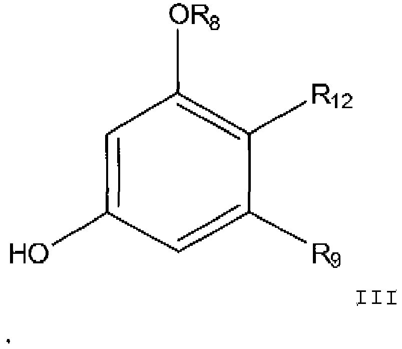

9. Process according to Claim 8, **characterized in that** the condensation is performed in the presence of acetals of N,N-dimethylformamide, for example N,N-dimethylformamide dineopentyl acetal or other water-releasing reagents, preferably propanephosphonic anhydride, these being added to the reaction solution as such or being applicable to a support material, for example aluminium oxide, in the presence of at least one of the following Brønsted or Lewis acids: perchloric acid, hydrohalic acids (HF, HCl, HBr, HI), sulphuric acid, hydrogensulphates, phosphoric acid and the acidic salts thereof, pyro- and polyphosphoric acids, organic carboxylic and sulphonic acids having one up to 30 carbon atoms and one or more acidic groups, and acidic groups bonded to polymeric supports, for example acidic ion exchangers and mixtures of the acids mentioned, for example formic acid, oxalic acid, trifluoroacetic acid, p-toluenesulphonic acid, cations of alkali metals and alkaline earth metals, and also transition metals; halogen compounds and other trivalent compounds of elements of the third main group, such as boron trifluoride and other boron-halogen compounds and complexes thereof, aluminium halides such as anhydrous aluminium chloride; salts and halogen compounds of transition metals such as titanium tetrachloride, zinc chloride, zinc trifluoromethanesulphonate; halogen compounds of elements of the fourth and fifth and sixth main groups, for example tin tetrachloride, phosphorus trichloride, phosphorus pentachloride, phosphorus oxychloride, antimony pentafluoride, thionyl chloride, sulphuryl chloride, either alone or in mixtures with other Brønsted or Lewis acids; positive centres bonded to polymeric frameworks, for example montmorillonite.

10. Process according to Claim 8 or 9, **characterized in that** the condensation is performed in at least one of the following water-miscible or water-immiscible solvents: hydrocarbons having up to 30 carbon atoms, halogenated hydrocarbons having up to 20 carbon atoms, for example dichloromethane or chloroform, ethers, for example 2-methoxytetrahydrofuran, alcohols, carboxylic acids having up to 16 carbon atoms, amides having up to 20 carbon atoms, esters having up to 60 carbon atoms, carbon dioxide, sulphur dioxide, water, water with a phase transfer catalyst, the acidic catalysts themselves, and mixtures of said solvents with one another.

11. Process according to either of Claims 1 and 2, **characterized in that** the compound of the formula la: 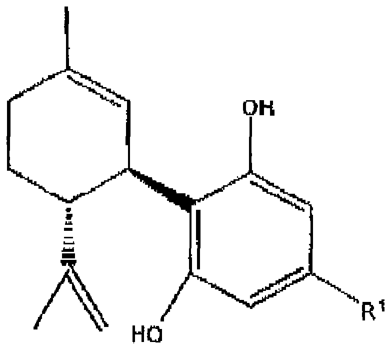 1a with R¹ = n-C₅H₁₁
is prepared by decarboxylating hydrolysis of the compound of the formula 16a 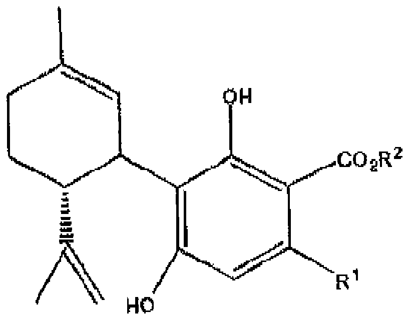 16a with R¹ = n-C₅H₁₁; R² = CH₃.

12. Process according to any of Claims 8 to 11, **characterized in that** the compound of the formula 16a 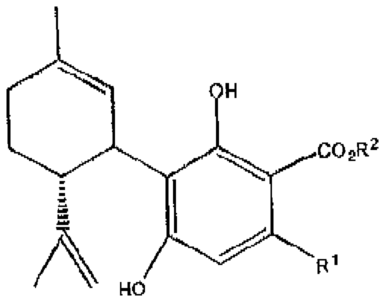 16a with R¹ = n-C₅H₁₁; R² = CH₃
is prepared by an acid-catalysed condensation of the compounds of the formulae 6a and 5a 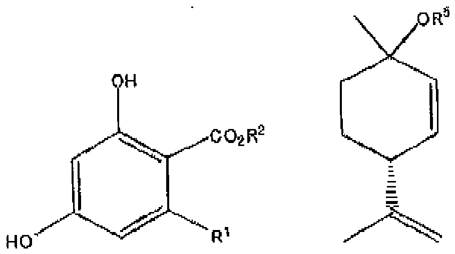 5a with R¹ = n-C₅H₁₁; R² = CH₃ 6a with R⁵ = H.

13. Process according to Claim 11, **characterized in that** (-)-delta-9-tetrahydrocannabinol (dronabinol, formula 2a) 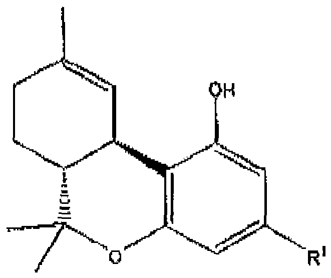 2a with R¹ = n-C₅H₁₁
is prepared in a downstream acid-catalysed isomerization step from the compound of the formula 1a.

14. Process according to any of the preceding claims, **characterized in that** the decarboxylating hydrolysis is preceded or followed by an acid-catalysed rearrangement.

15. Process according to Claim 14, **characterized in that** the rearrangement is an epimerisation.

16. Process for preparing compounds of the general formula Ia, Ib, 22 or 35 according to Claim 1 or 2, **characterized by** the following steps:
a) reacting compounds of the general formula III with a suitable unsaturated terpene according to Claim 8 in order to obtain a compound of the general formula IIa, IIb, IIc, 21 or 34 according to Claim 1 or 2;
b) performing a decarboxylating hydrolysis according to Claim 1 or 2; and
c) performing an acid-catalysed rearrangement, step c) being performable before step b).

## Revendications

1. Procédé pour la préparation d'un composé de formule générale Ia, Ib et leurs diastéréo-isomères 22, 35 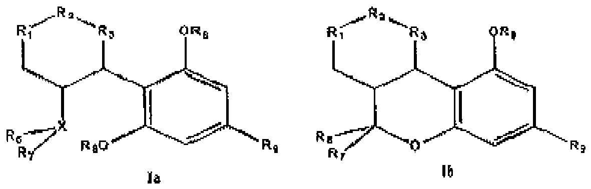 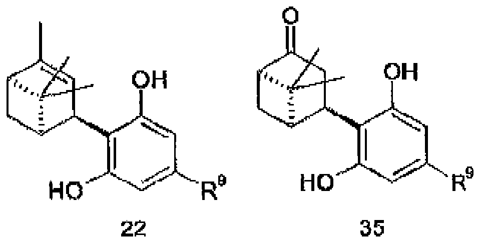 par saponification avec décarboxylation des composés de formule générale IIa, IIb, IIc ou 21 ou 34 sous pression dans un solvant à bas point d'ébullition 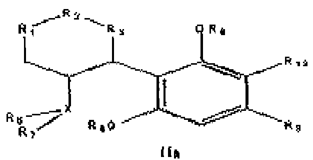 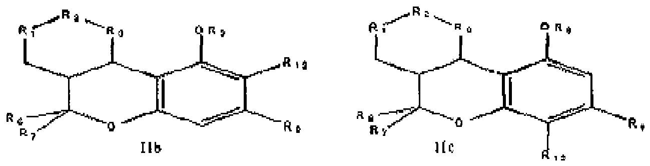 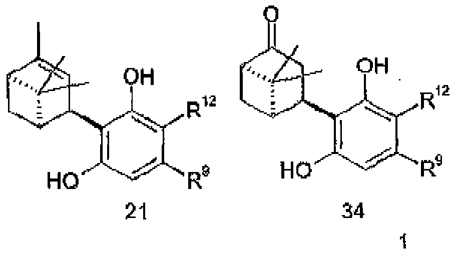 une des liaisons R₁-R₂ ou R₂-R₃ pouvant être une double liaison C-C, et
R₁ et R₃ représentant CH₂ ou CH, et
R₂ représentant soit C=O, soit un groupe R₁₀-C-R₁₁,
où
R₁₀ et R₁₁ représentent, indépendamment l'un de l'autre, soit H, soit un groupe alkyle inférieur en C₁-C₄, lorsqu'il n'existe pas de double liaison entre R₁ et R₃, soit, lorsqu'il existe une double liaison entre R₁ et R₃, un des groupes R₁₀ ou R₁₁ est supprimé et l'autre est tel que défini ci-dessus ;
X représente soit C, lorsque R₆ représente un groupe =CH₂ et R₇ représente un groupe CH₃, soit
X représente un groupe CR₄, R₄ représentant H ou un groupe alkyle inférieur, CH ou un groupe C-O-R₅ et R₅ représentant soit H, soit un groupe alkyle en C₁-C₁₈ soit un groupe de protection ;
R₆ et R₇ représentent un groupe CH₃ ou au moins un des groupes R₆ et R₇ représente un groupe CH₂= et l'autre représente un groupe CH₃,
R₈ représente un groupe alkyle en C₁-C₁₆, H ou un groupe de protection,
R₉ représente un groupe C₁-C₁₆ ou un groupe O-C₁-C₁₆, C₁-C₁₆ représentant une chaîne alkyle linéaire ou ramifiée qui présente, en un endroit quelconque, une ou plusieurs doubles ou triples liaisons ou qui peut présenter un ou plusieurs substituants tels que des atomes de deutérium ou d'halogène, des groupes phényle, phényle substitué, cycloalkyle, nitrile, alcoxy ou un groupe céto,
R₁₂ représente un groupe CO₂-R₁₃, et
R₁₃ représente un groupe alkyle en C₁-C₁₆ ou un groupe de protection,
la saponification avec décarboxylation étant réalisée dans un milieu alcalin.

2. Procédé pour la préparation d'un composé de formule générale Ia, Ib et leurs diastéréo-isomères 22 ou 35 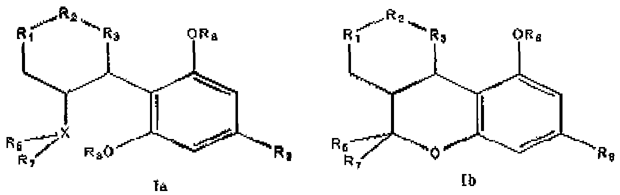 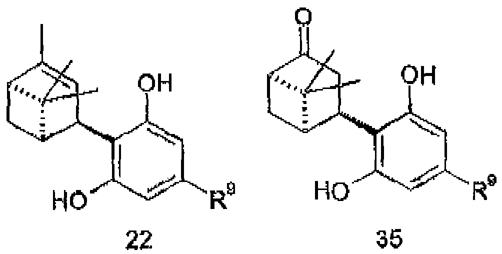 par saponification avec décarboxylation des composés de formule générale IIa, IIb, IIc ou 21 ou 34 à pression atmosphérique dans un solvant bouillant à plus de 100°C 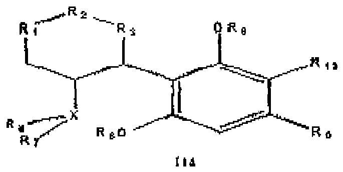 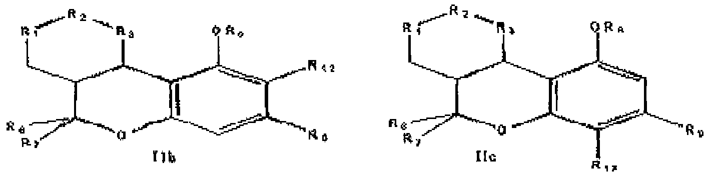 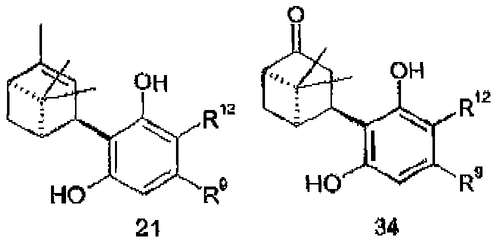 une des liaisons R₁-R₂ ou R₂-R₃ pouvant être une double liaison C-C, et
R₁ et R₂ représentant CH₂ ou CH, et
R₂ représentant soit C=O, soit un groupe R₁₀-C-R₁₁,
où
Rio et R₁₁ représentent, indépendamment l'un de l'autre, soit H, soit un groupe alkyle inférieur en C₁-C₄, lorsqu'il n'existe pas de double liaison entre R₁ et R₃, soit, lorsqu'il existe une double liaison entre R₁ et R₃, un des groupes R₁₀ ou R₁₁ est supprimé et l'autre est tel que défini ci-dessus ;
X représente soit C, lorsque R₆ représente un groupe =CH₂ et R₇ représente un groupe CH₃, soit
X représente un groupe CR₄, R₄ représentant H ou un groupe alkyle inférieur, CH ou un groupe C-O-R₅ et R₅ représentant soit H, soit un groupe alkyle en C₁-C₁₆ soit un groupe de projection ;
R₆ et R₇ représentent un groupe CH₃ ou au moins un des groupes R₆ et R₇ représente un groupe CH₂= et l'autre représente un groupe CH₃,
R₈ représente un groupe alkyle en C₁-C₁₆, H ou un groupe de protection,
R₉ représente un groupe C₁-C₁₆ ou un groupe O-C₁-C₁₆, C₁-C₁₆ représentant une chaîne alkyle linéaire ou ramifiée qui présente, en un endroit quelconque, une ou plusieurs doubles ou triples liaisons ou qui peut présenter un ou plusieurs substituants tels que des atomes de deutérium ou d'halogène, des groupes phényle, phényle substitué, cycloalkyle, nitrile, alcoxy ou un groupe céto,
R₁₂ représente un groupe CO₂-R₁₃, et
R₁₃ représente un groupe alkyle en C₁-C₁₆ ou un groupe de protection,
la saponification avec décarboxylation étant réalisée dans un milieu alcalin.

3. Procédé selon l'une quelconque des revendications 1 ou 2, **caractérisé en ce qu'**on utilise des catalyseurs de décarboxylation, par exemple des métaux de transition et leurs sels, de préférence des poudres d'acier noble ou des poudres d'argent.

4. Procédé selon la revendication 1, **caractérisé en ce qu'**on utilise, comme solvant, des alcools inférieurs comprenant jusqu'à cinq atomes de carbone, de l'ammoniaque ainsi que leurs mélanges les uns avec les autres et avec de l'eau.

5. Procédé selon la revendication 1, **caractérisé en ce que** le procédé est réalisé en milieu aqueux avec des catalyseurs de transfert de phase ou des émulsifiants.

6. Procédé selon la revendication 2, **caractérisé en ce qu'**on utilise au moins un des solvants suivants DMF, DMSO, sulfolane, furfurol, dihydrofurfurol et tétrahydrofurfurol, 2-méthoxytétrahydrofuranne, triamide de l'acide hexaméthylènephosphorique, acétamide, des amides comprenant jusqu'à 12 atomes de carbone, tétraméthylurée, éthylèneglycol, ainsi que ses monoéthers et bis-éthers, éthanolamine, éthylènediamine, propylèneglycol et ses éthers comprenant jusqu'à 20 atomes de carbone, glycérol et glycéroléthers comprenant jusqu'à 30 atomes de carbone, 1,2-butanediol, 1,3-butanediol, 1,4-butanediol, diéthylèneglycol et ses éthers, triéthylèneglycol et ses éthers, polyéthyléneglycol, polyvinylpyrrolidone avec ou sans eau.

7. Procédé selon l'une quelconque des revendications 1 à 6, **caractérisé en ce qu'**on utilise une ou plusieurs des bases suivantes pour la saponification alcaline : hydroxydes de métaux alcalins et alcalino-terreux ainsi que de sels d'ammonium quaternaire, carbonates, hydrogénocarbonates et carboxylates comprenant jusqu'à 30 atomes de carbone, phénolates, phosphates, phosphites, sulfures, hydrogénosulfures, mercaptides comprenant jusqu'à 30 atomes de carbone, sulfites, hydrogénosulfites, cyanures de métaux alcalins et de sels d'ammonium quaternaire comprenant 4 à 98 atomes de carbone, ammoniaque, alkylamines, arylamines, aminés hétérocycliques aromatiques ou non aromatiques, organiques, comprenant jusqu'à 36 atomes de carbone ainsi que leurs sels, borates, tétraborates.

8. Procédé selon l'une quelconque des revendications 1 ou 2, **caractérisé en ce que** le produit intermédiaire de type ester du composé de formule Ia, Ib, 22 ou 35 est préparé par une condensation catalysée d'un acide d'un précurseur terpénique insaturé approprié avec le composé de formule générale III, R₈, R₉ et R₁₂ étant tels que définis dans la revendication 1

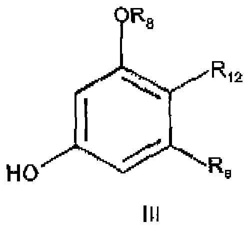

9. Procédé selon la revendication 8, **caractérisé en ce que** la condensation est réalisée en présence d'acétals de N,N-diméthylformamide, comme par exemple le N,N-diméthylformamide-dinéopentylacétal ou d'autres réactifs dissociant de l'eau, de préférence l'anhydride de l'acide propanephosphonique, ceux-ci étant ajoutés tels quels à la solution réactionnelle ou pouvant être appliqués sur un matériau support tel que par exemple l'oxyde d'aluminium, en présence d'au moins un des acides de Brönstedt ou de Lewis suivants : acide perchlorique, acides halogénohydriques (HF, HCl, HBr, HI), acide sulfurique, hydrogénosulfates, acide phosphorique et ses sels acides, acides pyrophosphorique et polyphosphorique, acides carboxyliques et sulfoniques organiques comprenant un jusqu'à 30 atomes de carbone et un ou plusieurs groupes acides, ainsi que groupes acides liés à un support polymère, tels que par exemple des échangeurs d'ions acides et des mélanges des acides mentionnés, par exemple acide formique, acide oxalique, acide trifluoroacétique, acide p-toluènesulfonique, cations de métaux alcalins et alcalino-terreux ainsi que métaux de transition ; composés halogénés et d'autres composés trivalents d'éléments du troisième groupe principal, tels que trifluorure de bore et d'autres composés de bore-halogène et leurs complexes, halogénures d'aluminium tels que le chlorure d'aluminium anhydre ; sels et composés halogénés de métaux de transition, tels que tétrachlorure de titane, chlorure de zinc, trifluorométhanesulfonate de zinc ; composés halogénés d'éléments du quatrième, cinquième et sixième groupe principal, par exemple tétrachlorure d'étain, trichlorure de phosphore, pentachlorure de phosphore, oxychlorure de phosphore, pentafluorure d'antimoine, chlorure de thionyle, chlorure de sulfuryle, soit seuls soit dans des mélanges avec d'autres acides de Brönstedt ou de Lewis ; centres positifs liés à des structures polymères, tels que par exemple montmorillonite.

10. Procédé selon la revendication 8 ou 9, **caractérisé en ce que** la condensation est réalisée dans au moins un des solvants suivants, miscibles ou non miscibles à l'eau : hydrocarbures comprenant jusqu'à 30 atomes de carbone, hydrocarbures halogénés comprenant jusqu'à 20 atomes de carbone tels que par exemple dichlorométhane ou chloroforme, éthers tels que par exemple 2-méthoxytétrahydrofuranne, alcools, acides carboxyliques comprenant jusqu'à 16 atomes de carbone, amides comprenant jusqu'à 20 atomes de carbone, esters comprenant jusqu'à 60 atomes de carbone, dioxyde de carbone, dioxyde de soufre, eau, eau avec un catalyseur de transfert de phase, les catalyseurs acides eux-mêmes ainsi que des mélanges des solvants mentionnés les uns avec les autres.

11. Procédé selon l'une quelconque des revendications 1 ou 2, **caractérisé en ce que** le composé de formule (1a) 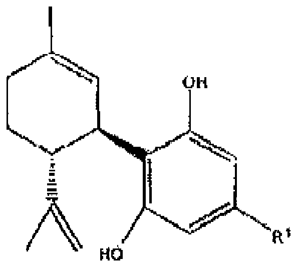 la avec R₁ = n-C₅H₁₁
est préparé par saponification avec décarboxylation du composé de formule 16a 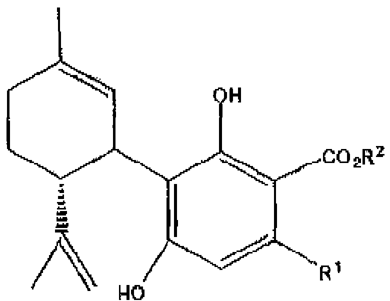 16a avec R₁ = n-C₅H₁₁ ; R₂ = CH₃.

12. Procédé selon l'une quelconque des revendications 8 à 11, **caractérisé en ce que** le composé de formule 16a 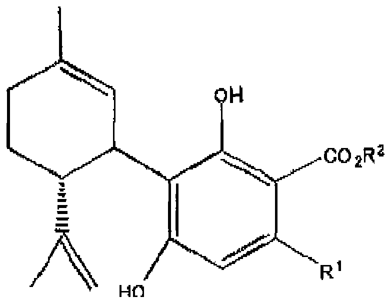 16a avec R₁ = n-C₅H₁₁ ; R₂ = CH₃.
est préparé par condensation catalysée par un acide des composés des formules 6a et 5a 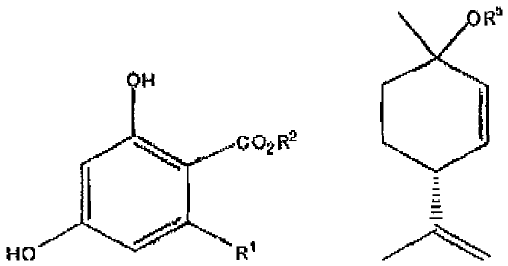 5a avec R₁ = n-C₆H₁₁ ; R₂ = CH₃ 6a avec R₅ = H.

13. Procédé selon la revendication 11, **caractérisé en ce qu'**on prépare, à partir du composé de formule 1a, dans une étape d'isomérisation consécutive catalysée par un acide, du (-)-delta-9-tétrahydrocannabinol (Dronabinol, formule 2a) 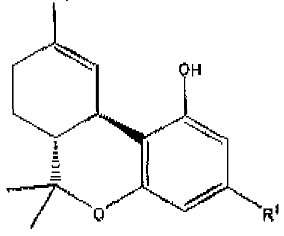 2a avec R₁ = n-C₅H₁₁.

14. Procédé selon l'une quelconque des revendications précédentes, **caractérisé en ce qu'**on réalise, avant ou après la saponification avec décarboxylation, une transposition catalysée par un acide.

15. Procédé selon la revendication 14, **caractérisé en ce que** la transposition est une épimérisation.

16. Procédé pour la préparation des composés de formule générale Ia, Ib, 22 ou 35 selon la revendication 1 ou 2, **caractérisé par** les étapes suivantes :
a) transformation de composés de formule générale III avec un terpène insaturé approprié selon la revendication 8, pour obtenir un composé de formule générale IIa, IIb, IIc, 21 ou 34 selon la revendication 1 ou 2 ;
b) réalisation d'une saponification avec décarboxylation selon la revendication 1 ou 2 ; et
c) réalisation d'une transposition catalysée par un acide,
l'étape c) pouvant être réalisée avant l'étape b).
